# EUROPEAN PATENT APPLICATION

(11) **EP 2 727 602 A1**
(43) Date of publication of application: **07.05.2014**
(21) Application number: 12190855.2
(22) Date of filing: 31.10.2012
(51) Int. Cl.: A61K 39/395, C07K 16/28

(54) **Method for preparation of a high concentration liquid formulation of an antibody**

(71) Applicant: Takeda GmbH, 78467 Konstanz (DE)
(72) Inventor: The designation of the inventor has not yet been filed
(74) Representative: Wild, Robert

(57) **Abstract**

The present invention provides a method for preparation of a high concentration liquid formulation (HCLF) of an antibody or a fragment thereof. The present invention also relates to a method for stabilizing an anti-CD20 antibody or a fragment thereof in a liquid pharmaceutical formulation. Furthermore, the present invention relates to a liquid pharmaceutical formulation of a veltuzumab antibody or a fragment thereof comprising at least 155 mg/mL of a veltuzumab antibody or a fragment thereof.

SEQUENCE LISTING

<110>  Nycomed GmbH - a Takeda Company

<120>  Method for preparation of a high concentration liquid formulation
        of an antibody

<130>  1714

<160>  2

<170>  PatentIn version 3.5

<210>  1
<211>  451
<212>  PRT
<213>  Artificial Sequence

<220>
<223>  Veltuzumab antibody heavy chain

<400>  1

Gln Val Gln Leu Gln Gln Ser Gly Ala Glu Val Lys Lys Pro Gly Ser
1               5                   10                  15

Ser Val Lys Val Ser Cys Lys Ala Ser Gly Tyr Thr Phe Thr Ser Tyr
            20                  25                  30

Asn Met His Trp Val Lys Gln Ala Pro Gly Gln Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Ala Asp Glu Ser Thr Asn Thr Ala Tyr
65                  70                  75                  80

Met Glu Leu Ser Ser Leu Arg Ser Glu Asp Thr Ala Phe Tyr Tyr Cys
                85                  90                  95

Ala Arg Ser Thr Tyr Tyr Gly Gly Asp Trp Tyr Phe Asp Val Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Arg Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210>  2
<211>  213
<212>  PRT
<213>  Artificial Sequence

<220>
<223>  Veltuzumab antibody light chain

<400>  2

Asp Ile Gln Leu Thr Gln Ser Pro Ser Ser Leu Ser Ala Ser Val Gly
1               5                   10                  15

Asp Arg Val Thr Met Thr Cys Arg Ala Ser Ser Ser Val Ser Tyr Ile
            20                  25                  30

His Trp Phe Gln Gln Lys Pro Gly Lys Ala Pro Lys Pro Trp Ile Tyr
        35                  40                  45

Ala Thr Ser Asn Leu Ala Ser Gly Val Pro Val Arg Phe Ser Gly Ser
    50                  55                  60

Gly Ser Gly Thr Asp Tyr Thr Phe Thr Ile Ser Ser Leu Gln Pro Glu
65                  70                  75                  80

Asp Ile Ala Thr Tyr Tyr Cys Gln Gln Trp Thr Ser Asn Pro Pro Thr
                85                  90                  95

Phe Gly Gly Gly Thr Lys Leu Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

## Description

The present invention provides a method for preparation of a high concentration liquid formulation (HCLF) of an antibody. The present invention also relates to a method for stabilizing an anti-CD20 antibody or a fragment thereof in a liquid pharmaceutical formulation. Furthermore, the present invention relates to a liquid pharmaceutical formulation of a veltuzumab antibody or a fragment thereof and the use of said pharmaceutical formulation as a medicament.

### Introduction

Therapeutic proteins have to fulfill a number of different criteria, e.g. as regards stability, administration and concentration in order to meet the requirements for regulatory approval as a drug. During manufacturing, storage and delivery chemical and/or physical degradation of therapeutic proteins such as antibodies may occur, which may lead to a loss of their pharmaceutical potency and increased risk of side effects, e.g. unwanted immune response.

Many therapeutic proteins need to be administered in high doses in order to achieve their desired therapeutic effect. Furthermore, high concentration formulations of therapeutic proteins are advantageous, as they may allow for a more convenient mode of administration of the therapeutic protein to the patient.

High concentrations of, e.g. at least 100 mg/mL, therapeutic protein are desirable as the volume necessary for the administration of the therapeutic dose decreases with increasing concentration. Smaller volumes provide the advantage that they may be injected via less invasive routes, such as subcutaneous injection instead of intravenous infusion, which is more convenient for the patient and potentially associated with less risks for side effects like infusion reactions. A further advantage provided by high concentration formulations is, that they may allow reducing the frequency of administration of the therapeutic protein to the patient.

However, the provision of high concentrated liquid formulations of therapeutic proteins (e.g. monoclonal antibodies) is challenging as the viscosity of the liquid formulation as well as the tendency of proteins to form aggregates may increase dramatically at higher concentrations. Aggregates can contain degradation products of the protein and may lead to unwanted side effects, e.g. triggering unwanted immune responses. In order to avoid stability problems such as the formation of aggregates, freeze drying may be used. Thus, many approved products with a concentration higher than 100 mg/mL are lyophilisates, which have to be reconstituted prior to administration. However, freeze drying is a time-consuming and costly process. Furthermore, reconstitution of lyophilisates is less convenient for patients and medicinal personal as well as error prone.

Various documents deal with highly concentrated antibody formulations and/or methods for providing the same:

WO 03/039485 describes stable liquid pharmaceutical formulations of antibodies, particularly of Daclizumab (an anti-IL2 receptor antibody), HAIL-12 (a humanized anti-IL12 antibody), HuEP5C7 (a humanized anti-selectin monoclonal antibody) and Flintozumab (a humanized anti-gamma interferone monoclonal antibody) having an antibody concentration of 50 mg/mL or greater. However, neither high concentration liquid formulations of a veltuzumab antibody nor methods for providing high concentration liquid formulations of antibodies according to the present invention are disclosed therein.

US 2012/0064086 also describes highly concentrated antibody formulations, in particular HCLFs of IgE antibodies, said HCLF having a reduced viscosity.

However, said document does not disclose antibody formulations comprising a veltuzumab antibody or the method for preparing a high concentration liquid formulation of an antibody as described herein.

WO 2011/029892 relates to highly concentrated, stable anti-CD20 antibody formulations. However, it does not describe a highly concentrated formulation of a veltuzumab antibody or the method for preparation of HCLFs as disclosed herein.

WO 2004/001007 describes a method for providing a composition of antibodies consisting essentially of an aqueous solution of antibodies and histidine or acetate buffer at a range from 2 mM to 48 mM. However, the method disclosed therein does not include an ultrafiltration step up to about 280 mg/mL.

US 2012/0064086 also discloses a method for producing a highly concentrated antibody formulation with reduced viscosity including three filtration steps (i.e. a first ultrafiltration step, a diafiltration step and a second ultrafiltration step), which are performed at elevated temperatures.

More general considerations regarding development of formulations for protein drugs are described in the minireview "Challenges in the development of high protein concentration formulations" (Steven J. Shire, Zahra Shahrokh, Jun Liu Journal of Pharmaceutical Sciences, Vol. 93, No. 6, June 2004, 1390-1402).

Hence, there is a need for high concentration liquid formulations of antibodies as well as for a method for providing such high concentration liquid formulations of antibodies.

### Summary of the Invention

One aspect of the invention relates to a method for preparation of a high concentration liquid formulation of an antibody having a concentration C^{H} of the antibody, comprising the steps of:
a) providing a solution containing the antibody in a starting concentration C^{s};
b) ultrafiltering the solution of step (a) in order to obtain a solution having an intermediate concentration C^{I} of the antibody, wherein C^{I} is at least about 260 mg/mL; and
c) diluting the solution of step (b) to a concentration C^{H} of the antibody in order to obtain the high concentration liquid formulation.

One embodiment of the invention relates to the method for preparation of a HCLF according to the invention, wherein the solution of step (a) further contains a buffering agent which is optionally an amino acid, such as histidine, specifically L-histidine.

Another embodiment of the invention relates to the method for preparation of a HCLF according to the invention, further comprising between step (a) and step (b) a step of diafiltering the solution of step (a) with a buffer solution, wherein the buffering agent is optionally an amino acid, such as histidine, specifically L-histidine.

A further embodiment of the invention relates to the method for preparation of a HCLF according to the invention, wherein the solution which is subjected to ultrafiltering in step (b) contains 40 mM histidine and has a pH value of 5.45.

One embodiment of the invention relates to the method for preparation of a HCLF according to the invention, wherein the solution which is subjected to ultrafiltering in step (b) is essentially free of or does not contain a tonicity modifying agent such as sucrose.

Another embodiment of the invention relates to the method for preparation of a HCLF according to the invention, wherein the solution which is subjected to ultrafiltering in step (b) is essentially free of or does not contain a surfactant such as polysorbate.

One further embodiment of the invention relates to the method for preparation of a HCLF according to the invention, wherein the antibody is an anti-CD20 antibody and/or an IgG antibody, optionally the antibody is a veltuzumab antibody.

One embodiment of the invention relates to the method for preparation of a HCLF according to the invention, wherein the high concentration liquid formulation has an antibody concentration C^{H} of at least 155 mg/mL.

One embodiment of the invention relates to the method for preparation of a HCLF according to the invention, wherein step (b) is performed in an ultrafiltration device and further comprising between step (b) and step (c) a step of flushing the ultrafiltration device with a buffer solution, wherein the buffering agent is optionally an amino acid, such as histidine, specifically L-histidine.

A further aspect of the invention relates to a method for stabilizing an anti-CD20 antibody or a fragment thereof in a liquid pharmaceutical formulation in a concentration of at least 155 mg/mL by combining the antibody or fragment thereof which has not been freeze-dried with an aqueous solution comprising an amino acid, optionally histidine, specifically L-histidine.

One embodiment of the invention relates to the method for stabilizing an anti-CD 20 antibody or a fragment thereof, wherein the anti-CD20 antibody is a veltuzumab antibody.

Another aspect of the present invention relates to a liquid pharmaceutical formulation of a veltuzumab antibody or a fragment thereof comprising at least 155 mg/mL veltuzumab antibody or a fragment thereof and an amino acid, optionally histidine, specifically L-histidine.

One embodiment of the invention relates to the liquid pharmaceutical formulation, wherein the concentration of the veltuzumab antibody or a fragment thereof is at least 175 mg/mL, preferably at least 190 mg/mL.

Another embodiment of the invention relates to the liquid pharmaceutical formulation, wherein the concentration of the veltuzumab antibody or a fragment thereof is at least 200 mg/mL, preferably at least 220 mg/mL.

A further embodiment of the invention relates to the liquid pharmaceutical formulation, wherein the concentration of the amino acid, optinally histidine, specifically L-histidine, is in the range of 1 mM to 100 mM.

Another embodiment of the invention relates to the liquid pharmaceutical formulation, wherein the concentration of the amino acid, optionally histidine, specifically L-histidine, is in the range of 10 mM to 60 mM, optionally in the range of 20 mM to 50 mM.

One embodiment of the invention relates to the liquid pharmaceutical formulation, further comprising a surfactant, optionally a nonionic surfactant.

One embodiment of the invention relates to the liquid pharmaceutical formulation, wherein the surfactant, optionally the nonionic surfactant, is a polysorbate, optionally polysorbate 20 or polysorbate 80.

A further embodiment of the invention relates to the liquid pharmaceutical formulation, wherein the surfactant, optionally the nonionic surfactant, is present in a concentration of at least 0.01 mg/mL, optionally in the range of 0.1 mg/mL to 1 mg/mL.

One embodiment of the invention relates to the liquid pharmaceutical formulation, further comprising a tonicity modifying agent.

Another embodiment of the invention relates to the liquid pharmaceutical formulation, wherein the tonicity modifying agent is sorbitol and/or mannitol, optionally the concentration of the tonicity modifying agent is in the range of 5.5 mM to 550 mM.

One embodiment of the invention relates to the liquid pharmaceutical formulation having a pH value in the range of 4.8 to 7.0, optionally a pH value of 5.5 ± 0.3.

One embodiment of the invention relates to the liquid pharmaceutical formulation comprising
a) at least 160 mg/mL veltuzumab antibody or a fragment thereof;
b) 220 mM sorbitol;
c) 30 mM histidine;
d) 0.2 mg/mL polysorbate 20; and
having a pH value in the range of 5.0 to 6.0, optionally 5.5±0.3.

Another embodiment of the invention relates to the liquid pharmaceutical formulation for use as a medicament.

One further embodiment of the invention relates to the liquid pharmaceutical formulation, wherein the medicament is for subcutaneous administration.

One embodiment of the invention relates to the liquid pharmaceutical formulation, for use in the treatment of cancer or a non-malignant disease, optionally an inflammatory or autoimmune disease, including Class III autoimmune diseases.

Another embodiment of the invention relates to the liquid pharmaceutical formulation, for use in the treatment of a disease selected from the group consisting of Burkitt Lymphoma, Epstein-Barr Virus Infections, B-Cell Leukemia, Chronic Lymphocytic B-Cell Leukemia, Acute Lymphoblastic Leukemia, Lymphoid Leukemia, Prolymphocytic Leukemia, Hairy Cell Leukemia, Multiple Myeloma, B-Cell Lymphoma, Marginal Zone B-Cell Lymphoma, Follicular Lymphoma, Diffuse Large B-Cell Lymphoma, Immunoblastic Large-Cell Lymphoma, Mantle-Cell Lymphoma, Non-Hodgkin Lymphoma, Lymphomatoid Granulomatosis, Plasma Cell Neoplasms, Precursor Cell Lymphoblastic Leukemia-Lymphoma, Tumor Virus Infections, Waldenstrom Macroglobulinemia, Immunoproliferative Disorders, Prolymphocytic Lymphoma, Diffuse Large B-Cell Lymphoma, Immunoblastic Large-Cell Lymphoma, Mantle-Cell Lymphoma, Lymphomatoid Granulomatosis, Lymphoproliferative Disorders, Paraproteinemias, Precursor Cell Lymphoblastic Leukemia-Lymphoma, Thrombocytopenic Purpura, Idiopathic Thrombocytopenic Purpura, Blood Coagulation Disorders, Blood Platelet Disorders, Blood Protein Disorders, Hematologic Diseases, Hemorrhagic Disorders, Hemostatic Disorders, Lymphatic Diseases, Purpura, Thrombocytopenia, Thrombotic Microangiopathies, Haemostatic Disorders, Vascular Diseases, Systemic lupus erythematosus (SLE), Lupus (e.g. nephritis, non-renal, discoid, alopecia), Juvenile onset diabetes, Multiple sclerosis, Rheumatoid Arthritis, Rheumatic Diseases, Connective Tissue Diseases, Herpesviridae Infections, and/or DNA Virus Infections.

### Brief Description of the Drawings

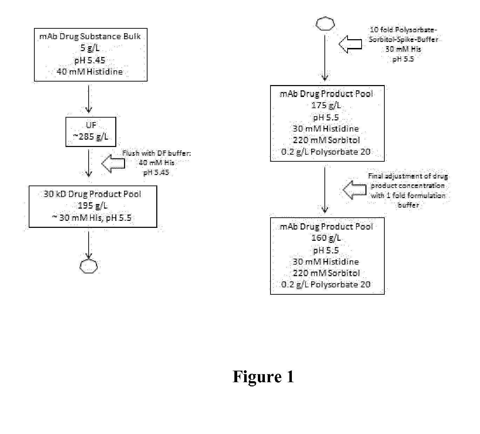

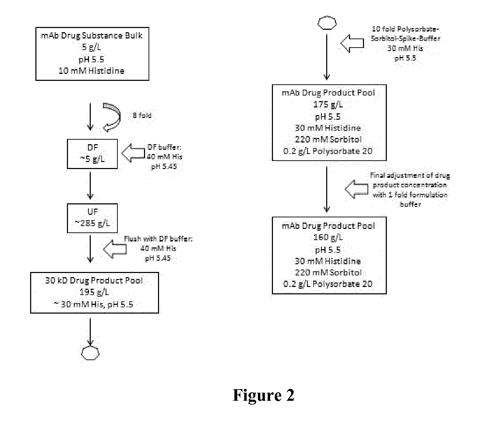

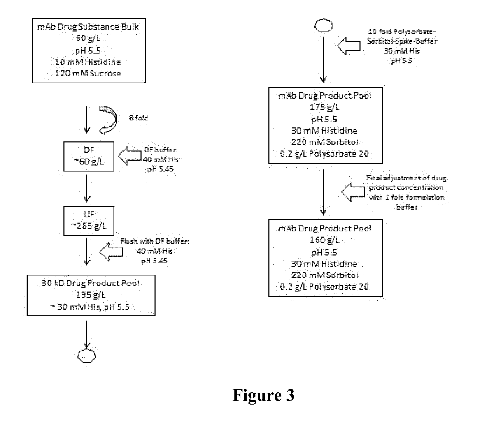

**Figure 1** shows an exemplary method according to the invention for providing a HCLF without an upstream diafiltration (DF) step. The final product in this example has a pH value of 5.5 and comprises 30 mM histidine (His). In order to compensate for the Donnan effect during ultra filtration (UF), a higher histidine concentration and a lower pH value can be adjusted in the starting material. Due to the so-called Donnan effect, charged molecules which are able to pass the UF membrane are either depleted (such as in the present case) or concentrated in the solution depending on the sign of charge in relation to the charge of the protein which cannot pass through the membrane. The depletion of positively charged histidine goes along with a pH shift in the solution. As a starting material a bulk drug substance comprising 5 g/L veltuzumab and 40 mM histidine and having a pH value of 5.45 can be used. The starting material is subjected to an ultrafiltration step. In the ultrafiltration step veltuzumab is concentrated up to a concentration of approximately 285 g/L. After this concentration has been reached, the ultrafiltration device can be flushed with a diafiltration buffer comprising 40 mM histidine and having a pH of 5.45. This may allow maximizing the yield of antibody obtained by the method described herein. The thereby obtained drug product pool in this example has a concentration of 195 g/L veltuzumab, a pH value of 5.5 and comprises approximately 30 mM histidine. Subsequently, a further dilution step with a so-called 10 fold polysorbate-sorbitol spike buffer solution comprising 30 mM histidine, 2.2 M sorbitol, 2 g/L polysorbate and having a pH value of 5.5 can be performed. The polysorbate-sorbitol spike buffer solution comprises the 10 fold concentration of the desired concentration of polysorbate and sorbitol in the final product. The resulting drug product pool in this example comprises 175 g/L veltuzumab, 30 mM histidine, 220 mM sorbitol, 0.2 g/L polysorbate 20 and has a pH value of 5.5. Afterwards a final adjustment of the drug product to the desired concentration can be performed via dilution with a so called 1-fold formulation buffer solution comprising 30 mM histidine, 220 mM sorbitol and 0.2 g/L polysorbate 20 and having a pH value of 5.5. The formulation buffer solution comprises a similar concentration of polysorbate and sorbitol as the final drug product pool (i.e. a 1-fold concentration). The final drug product pool in this example comprises 160 g/L veltuzumab, 30 mM histidine, 220 mM sorbitol and 0.2 g/L polysorbate 20 and has pH value of 5.5.
   Another exemplary method of the invention disclosed in **figure 2** differs from the method as disclosed in figure 1 in that the starting material comprises 5 g/L veltuzumab and 10 mM histidine and has a pH value of 5.5. Prior to ultrafiltration a diafiltration step is performed, wherein the buffer is exchanged with eight fold volume by using a diafiltration buffer solution comprising 40 mM histidine and having a pH of 5.45, thus equilibrating the final solution by removing the former buffer composition. The subsequently performed steps are in accordance with the steps performed in the exemplary method shown in figure 1.
   The exemplary method of the invention disclosed in **figure 3** differs from the method as disclosed in figure 1 in that the starting material comprises 60 g/L veltuzumab, 120 mM sucrose and 10 mM histidine and has a pH value of 5.5. Prior to ultrafiltration a diafiltration step is performed. Thereby sucrose is removed. Then the buffer is exchanged 8 fold by using a diafiltration buffer solution comprising 40 mM histidine and having a pH of 5.45. The subsequently performed steps are in accordance with the steps performed in the method shown in figure 1.

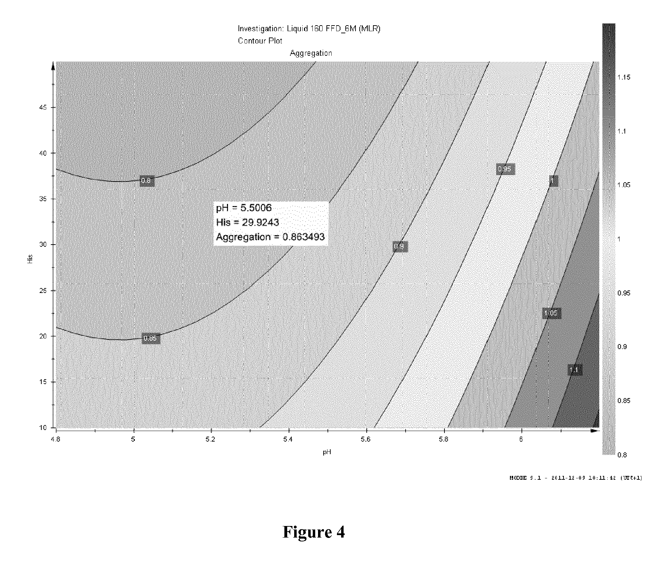

**Figure 4** shows a Response Contour Plot for formulation optimization; aggregation (in % of absolute antibody amount) after six months at 2-8 °C.

### Definitions

Where the term "comprise" or "comprising" is used in the present description and claims, it does not exclude other elements or steps. For the purpose of the present invention, the term "consisting of" is considered to be an optional embodiment of the term "comprising of". If hereinafter a group is defined to comprise at least a certain number of embodiments, this is also to be understood to disclose a group which optionally consists only of these embodiments.

Where an indefinite or a definite article is used when referring to a singular noun e.g. "a" or "an", "the", this includes a plural form of that noun unless specifically stated. *Vice versa,* when the plural form of a noun is used it refers also to the singular form. For example, when anti-CD20 antibodies are mentioned, this is also to be understood as a single anti-CD20 antibody.

Furthermore, the terms first, second, third or (a), (b), (c) and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. However, in a specific embodiment of the invention, the method steps (a), (b) and (c), optionally including any intermediate steps defined herein, are performed in chronological order.

In the context of the present invention any numerical value indicated is typically associated with an interval of accuracy that the person skilled in the art will understand to still ensure the technical effect of the feature in question. As used herein, the deviation from the indicated numerical value is in the range of ± 10%, and preferably of ± 5%. The aforementioned deviation from the indicated numerical interval of ± 10%, and preferably of ± 5% is also indicated by the terms "about" and "approximately" used herein with respect to a numerical value.

As used herein "essentially free of" means, that the component so identified is not being present in an amount that is detectable under typical conditions used for its detection. Furthermore, "essentially free of" also includes that the component is not present in an amount which adversely affects the desired properties of a composition or formulation, i.e. that the compound so-identified is present in an negligible amount.

As used herein the term "liquid formulation" refers to a formulation in a liquid state and includes liquid formulations originally in a liquid state as well as resuspended lyophilized/freeze-dried formulations, i.e. so called reconstituted solutions. However, it is not necessary for stability reasons that the liquid formulation disclosed herein be lyophilized/freeze-dried or have its state changed by other methods, e.g. by spray drying. In one embodiment of the invention, the term "liquid formulation" does not include a resuspended or reconstituted lyophilized/freeze-dried formulation.

In the context of the present invention the term "antibody" relates to full length antibodies, human antibodies, humanized antibodies, fully human antibodies, genetically engineered antibodies (e.g. monoclonal antibodies, polyclonal antibodies, chimeric antibodies, recombinant antibodies) and multispecific antibodies, as well as to fragments of such antibodies retaining the characteristic properties of the full length antibody. In one embodiment, the antibody is a humanized antibody. A "humanized antibody" is an antibody which has been modified in order to provide an increased similarity to antibodies produced in humans, e.g. by grafting a murine CDR into the framework region of a human antibody.

The term "antibody fragment" relates to a part of a full length antibody binding with the same antigen as the full length antibody. In particular, it relates to a pharmaceutically active fragment of an antibody. This part of a full length antibody may be at least the antigen binding portion or at least the variable region thereof. Genetically engineered proteins acting like an antibody are also included within the meaning of antibody fragment as used herein. Such genetically engineered antibodies may be scFv, i.e. a fusion protein of a heavy and a light chain variable region connected by a peptide linker. Further exemplary antibody fragments are Fab, Fab', F(ab')₂, and Fv.

Anti-CD 20 antibody as defined herein denotes any antibody or fragment thereof that binds specifically to the CD 20 antigen (also known as CD 20 (Cluster of differentiation 20), B-lymphocyte antigen CD 20, B-lymphocyte surface antigen B1, Leu-16 or Bp35). This includes anti-CD 20 antibodies having marketing approval, anti-CD 20 antibodies currently studied in clinical trials and/or any other compound which binds specifically to the CD 20 antigen. The CD 20 antigen as used herein relates to any variants, isoforms and species homologs of human CD 20. Anti-CD 20 antibodies as used herein, relates to type I anti-CD 20 antibodies as well as to type II anti-CD 20 antibodies, which differ in their mode of CD 20 binding and their biological activities. Examples of anti-CD20 antibodies are Veltuzumab, Rituximab, Ocrelizumab, Ofatumumab, Y⁹⁰ Ibritumomab tiuxetan, I¹³¹ tositumab, TRU-015, AME-133v, PRO131921 humanized, GA101, 1F5 IgG2a, HI47 IgG3, 2C6 IgG1, 2H7 IgG1, AT80 IgG1, 11B8 IgG1, humanized B-Ly1 antibody IgG1 and Aftuzumab (HuMab<CD20>). Particularly, the anti-CD20 antibody is veltuzumab or a fragment thereof.

Veltuzumab is a monoclonal humanized anti-CD 20 antibody of the class IgG1/κ composed of mature heavy and light chains of 451 and 213 amino acid residues, respectively. Veltuzumab has the amino acid sequence set out below:
Heavy Chain (SEQ ID NO: 1) 
1 QVQLQQSGAE VKKPGSSVKV SCKASGYTFT SYNMHWVKQA PGQGLEWIGA
51 IYPGNGDTSY NQKFKGKATL TADESTNTAY MELSSLRSED TAFYYCARST
101 YYGGDWYFDV WGQGTTVTVS SASTKGPSVF PLAPSSKSTS GGTAALGCLV
151 KDYFPEPVTV SWNSGALTSG VHTFPAVLQS SGLYSLSSVV TVPSSSLGTQ
201 TYICNVNHKP SNTKVDKRVE PKSCDKTHTC PPCPAPELLG GPSVFLFPPK
251 PKDTLMISRT PEVTCVVVDV SHEDPEVKFN WYVDGVEVHN AKTKPREEQY
301 NSTYRVVSVL TVLHQDWLNG KEYKCKVSNK ALPAPIEKTI SKAKGQPREP
351 QVYTLPPSRE EMTKNQVSLT CLVKGFYPSD IAVEWESNGQ PENNYKTTPP
401 VLDSDGSFFL YSKLTVDKSR WQQGNVFSCS VMHEALHNHY TQKSLSLSPG
451 K
Light Chain (SEQ ID NO: 2)

1 DIQLTQSPSS LSASVGDRVT MTCRASSSVS YIHWFQQKPG KAPKPWIYAT
51 SNLASGVPVR FSGSGSGTDY TFTISSLQPE DIATYYCQQW TSNPPTFGGG
101 TKLEIKRTVA APSVFIFPPS DEQLKSGTAS VVCLLNNFYP REAKVQWKVD
151 NALQSGNSQE SVTEQDSKDS TYSLSSTLTL SKADYEKHKV YACEVTHQGL
201 SSPVTKSFNR GEC

Anti-CD20 antibodies according to the present invention may be monoclonal or polyclonal antibodies. Monoclonal antibodies are monospecific antibodies (i.e. binding to the same epitope) derived from a single cell line. Hence, monoclonal antibodies are, except for variants arising during their production, substantially identical antibodies. In contrast thereto, polyclonal antibodies relates to a variety of antibodies directed to different epitopes of an antigen. Methods for production of monoclonal and polyclonal antibodies are known in the art and include e.g. the hybridoma technology and recombinant DNA methods. In one embodiment of the invention the anti-CD20 antibody is a monoclonal antibody.

According to the present invention "IgG antibody" relates to a therapeutically useful antibody or fragment thereof falling within the IgG class (isotype) of antibodies and having a gamma-type (y) heavy chain. This includes an antibody of any subtype of the IgG class known in the art, i.e. IgG1, IgG2, IgG3 or IgG4. In one embodiment, the antibody is an IgG1 antibody. It is understood herein, that IgG antibodies also includes antibodies binding specifically to the CD 20 antigen and *vice versa.* One exemplary IgG antibodies of the present invention is veltuzumab. It is understood that "binds specifically" or "specifically binding" relates to an antibody having a binding affinity to the CD 20 antigen as defined herein of ≤10⁻⁹ mol/1, particularly of ≤10⁻¹⁰ mol/1. Methods for determining the binding affinity of antibodies to antigens are known in the art and include e.g. the use of surface plasmon resonance.

"Surfactant" as used herein relates to a surface-active agent, which is pharmaceutically acceptable. Surfactants can protect the therapeutic protein (e.g. the antibody as defined herein) from stress such interfacial tension between two liquids or between a liquid and a solid and/or can reduce the tendency to aggregate or the formation of particulates. Pharmaceutically acceptable surfactants include non-ionic surfactants, e.g. polysorbates and poloxamers but are not limited thereto. Exemplary surfactants useful in the present invention are polyoxyethylen-polyoxypropylene copolymers (e.g. Poloxamer 188), polyoxyethylene alkyl ethers and polysorbates (e.g., Polysorbate 20, Polysorbate 80). It is understood that also combinations of surfactants, e.g. combinations of the aforementioned surfactants may be used.

In the context of the present invention "pharmaceutically acceptable" relates to any compound which may be used in a pharmaceutical composition without causing any undesired effects (such as negative side effects) in a patient to which the composition is administered.

"Tonicity modifying agent" as used herein refers to any pharmaceutically acceptable agent suitable to provide an isotonic formulation. Isotonic formulations are formulations having the same tonicity (i.e. solute concentration) as the formulation to which they are compared (e.g. whole blood, blood serum or physiologic salt solution). Suitable tonicity modifying agents within the meaning of the present invention include NaCl, potassium chloride, glycine, glycerol, salts, amino acids, sugar alcohols (e.g. sorbitol and mannitol) and sugars (e.g. glucose, sucrose, trehalose, and glucose). Optionally, the tonicity modifying agent is a sugar or a sugar alcohol, in particular sorbitol, sucrose and/or mannitol. In a specific embodiment the tonicity modifying agent is a sugar, in particularl sorbitol. It is understood that also combinations of tonicity modifying agents, in particular combinations of the aforementioned tonicity modifying agents may also be used. In particular, it is understood that the tonicity modifying agent may be used in order to provide a physiologic tonicity in the formulation, i.e. a formulation having essentially the same tonicity as human blood. Such formulations will generally have an osmolarity of approximately 300 mOsm/kg, particularly 310 mOsm/kg.

As used herein the term "histidine" (His) specifically includes L-histidine unless otherwise specified.

"Patients" as used herein relates to any mammalian, in particular a human, suffering from any of the diseases or conditions mentioned herein, having been diagnosed with any of the diseases or conditions mentioned herein, being predisposed to any of the diseases or conditions mentioned herein or susceptible to any of the diseases or conditions mentioned herein.

According to the present invention "treatment" or "therapy" relates to therapeutic and/or preventive treatment of patients as defined herein.

"Room temperature" as used herein denotes a temperature in the range of 18°C to 25°C, in particular about 19°C to about 22°C. Specifically, room temperature denotes 20°C or 25°C.

Further definitions of the terms will be given below in the context of which the terms are used.

### Detailed Description of the Invention

The present invention provides a method for preparation of a high concentration liquid formulation (HCLF) of an antibody or a fragment thereof. The method according to the present invention allows to provide a HCLF and to obtain a relatively high yield of antibody.

In one of its embodiments, the HCLF has a concentration C^{H} of the antibody or a fragment thereof of at least 150 mg/mL, at least 155 mg/mL, at least 160 mg/mL, at least 175 mg/mL, at least 180 mg/mL, at least 185 mg/mL, at least 190 mg/mL, at least 195 mg/mL, at least 200 mg/mL, at least 220 mg/mL, at least 240 mg/mL, at least 260 mg/mL, at least 280 mg/mL, at least 285 mg/mL, or at least 290 mg/mL. In one specific embodiment, the HCLF has a concentration C^{H} of the antibody of at least 155 mg/mL. In another specific embodiment, the HCLF has a concentration C^{H} of the antibody or a fragment thereof of at least 160 mg/mL. In another specific embodiment, the HCLF has a concentration C^{H} of the antibody or a fragment thereof of at least 175 mg/mL. In a further specific embodiment, the HCLF has a concentration C^{H} of the antibody or a fragment thereof of at least 200 mg/mL. In another embodiment of the invention, the HCLF of the present invention has a concentration C^{H} of the antibody or a fragment thereof of 160-290 mg/mL, 160-250 mg/mL, 160-220 mg/mL, 160-200 mg/mL, or 160-175 mg/mL.

The present invention provides a method for preparation of a high concentration liquid formulation of an antibody or a fragment thereof having a concentration C^{H} of the antibody, comprising the steps of:
a) providing a solution containing the antibody in a starting concentration C^{s};
b) ultrafiltering the solution of step (a) in order to obtain a solution having an intermediate concentration C^{I} of the antibody, wherein C^{I} is at least about 260 mg/mL; and
c) diluting the solution of step (b) to a concentration C^{H} of the antibody in order to obtain the high concentration liquid formulation.

In a specific embodiment, the method is for preparation of a HCLF of an anti-CD 20 antibody and/or an IgG antibody. In another specific embodiment, the method is for preparation of a HCLF of an anti-CD 20 antibody and/or combinations of an anti-CD 20 antibody with a further active ingredient. In another specific embodiment, the method is for preparation of a HCLF of a humanized, monoclonal anti-CD 20 antibody and/or combinations of a humanized, monoclonal anti-CD 20 antibody with a further active ingredient. In a specific embodiment, the method is for preparation of a HCLF of an IgG antibody and/or combinations of an IgG antibody with a further active ingredient. In another embodiment of the invention, the method is for preparation of a HCLF of a veltuzumab antibody and/or combinations of a veltuzumab antibody with a further active ingredient.

In a specific embodiment, the method is for preparation of a HCLF of an anti-CD 20 antibody fragment and/or an IgG antibody fragment. In another specific embodiment, the method is for preparation of a HCLF of an anti-CD 20 antibody fragment and/or combinations of an anti-CD 20 antibody fragment with a further active ingredient. In another specific embodiment, the method is for preparation of a HCLF of a humanized, monoclonal anti-CD 20 antibody fragment and/or combinations of a humanized, monoclonal anti-CD 20 antibody fragment with a further active ingredient. In a specific embodiment, the method is for preparation of a HCLF of an IgG antibody fragment and/or combinations of an IgG antibody fragment with a further therapeutic agent. In another embodiment of the invention, the method is for preparation of a HCLF of a veltuzumab antibody fragment and/or combinations of a veltuzumab antibody fragment with a further therapeutic agent.

The further therapeutic agent may be administered separately, concurrently or sequentially with the formulation according to the present invention. This further therapeutic agent may be any therapeutic agent suitable in the treatment of any of the diseases mentioned herein. Examples of such further therapeutic agents are e.g. cytotoxic agents, anti-angiogenic agents, corticosteroids, antibodies, anti-inflammatory agents, chemotherapeutics, hormones and immunomodulators.

Examples of such therapeutic agents can be selected from the group comprising antimitotic, antikinase, alkylating, antimetabolite, antibiotic, alkaloid, antiangiogenic, apoptotic agents and combinations thereof. Chemotherapeutic drugs, for example, can be selected from the group comprising drugs such as vinca alkaloids, anthracyclines, epidophyllotoxin, taxanes, antimetabolites, alkylating agents, antikinase agents, antibiotics, Cox-2 inhibitors, antimitotics, antiangiogenic and apoptotoic agents, particularly doxorubicin, methotrexate, taxol, CPT-II, camptothecans, and others from these and other classes of anticancer agents.

Further examples of such therapeutic agents are described e.g. in the international patent application WO2003/068821, whose content is incorporated herein and referenced herewith.

In one embodiment of the invention, starting concentration C^{S} of the antibody or a fragment thereof as defined herein denotes an antibody concentration of at least 2 g/L, at least 5 g/L, at least 10 g/L, at least 20 g/L, at least 30 g/L, at least 40 g/L, at least 50 g/L, at least 60 g/L, at least 70 g/L, at least 80 g/L, at least 90 g/L, or at least 100 g/L. In one embodiment, C^{s} of the antibody or fragment thereof in the solution provided in step a) is at least 5 g/L. In another embodiment, C^{S} of the antibody or fragment thereof in the solution provided in step a) is at least 60 g/L.

In one embodiment of the invention, the solution provided in step a) of the method according to the invention which optionally also is the solution subjected to ultrafiltration in step b) of the method according to the invention is a solution further containing a buffering agent. In a specific embodiment, the solution provided in step a) of the invention is a composition containing an anti-CD20 antibody or a fragment thereof, in particular a veltuzumab antibody, and a buffering agent. In a specific embodiment, the solution provided in step a) of the invention is a solution containing an IgG antibody or fragment thereof and a buffering agent.

As used herein a buffering agent denotes a weak acid or base allowing to maintain the pH-value in a solution at a nearly constant level, such as phosphate, acetate (e.g., sodium acetate), succinate (such as sodium succinate), gluconate, glutamate, citrate, other organic acid buffers ,buffering agents comprising amino acids such as alanine, arginine, asparagine, aspartic acid, cysteine, glutamic acid, glutamin, glycine, histidine, isoleucine, leucine, lysine, methionine, phenylalanine, proline, serine, threonine, tryptophan, tyrosine, valine, specifically buffering agents comprising histidine. Buffering agents comprising amino acids are known to the person skilled in the art and can be chosen according to their desired buffering properties. Exemplary buffering agents comprising histidine are histidine, histidine chloride, histidine hydrochloride monohydrate, histidine acetate, histidine phosphate, histidine sulfate and mixtures thereof. Particularly, the buffering agent is histidine and/or histidine hydrochlorid monohydrate. Optionally, the buffering agent is histidine. It is understood, that any specification mentioned herein for histidine also relates to the exemplary buffering agents specifically the histidine buffering agents mentioned above. A further definition of buffering agents comprising histidine is provided below.

In one embodiment of the invention, the histidine concentration in the solution provided in step a) of the method according to the invention is 40 mM. In a further embodiment of the invention, the solution provided in step a) of the invention has a pH value of 5.45. Specifically, the solution provided in step a) of the method according to the invention has a pH-value of 5.45 and a histidine concentration of 40 mM.

The solution containing the antibody or a fragment thereof may be provided by any method known to the person skilled in the art. In one embodiment, a bulk drug substance comprising the antibody or a fragment thereof as defined herein is used as a starting material in order to provide the composition containing the antibody and e.g. histidine. Bulk drug substances include e.g. those provided by Immunomedics and Boehringer Ingelheim, both of which contain the anti-CD 20 antibody veltuzumab. This starting material may be subsequently purified in order to remove undesired ingredients and to provide the antibody or a fragment thereof in a starting concentration C^{s} and, optionally a buffering agent, e.g. L-histidine. Such undesired ingredients may include, e.g. sucrose, which can lead to a rapid increase of viscosity in the composition during the subsequently performed ultrafiltration step. High viscosity could lower the antibody concentrations achieved in the end product and generally hampers various process steps such as filtration. In the final product high viscosity could have a negative impact on the administration (e.g. the syringe ability).

Purification may be performed by any method known in the art such as filtration and centrifugation. Filtration includes any conventional filtration method for static filtration (e.g. vacuum filtration) and dynamic filtration (e.g. tangential flow filtration, diafiltration). In one embodiment of the method according to the invention, purification is performed by means of diafiltration (DF) or dialysis. In a specific embodiment purification is performed by means of diafiltration. In another specific embodiment purification is performed by means of dialysis.

As used herein "dialysis" relates to any process wherein molecules are purified by means of their different diffusion rates through a semipermeable membrane. Suitable methods for purification by means of dialysis are known to the person skilled in the art.

As used herein, "diafiltration" relates to a process using an ultrafiltration membrane in order to remove, replace, or lower the concentration of solvents from compositions comprising proteins, peptides, nucleic acids, or other biomolecules, in particular antibodies. Diafiltration provides the advantage that purification, concentration and, if necessary, buffer exchange may be performed in a single operation unit. Diafiltration devices are known in the art and include e.g. the Tangential Flow Filtration (TFF) Unit Minim II provided by Pall.

Hence, in one embodiment of the invention the method comprises between step a) and step b) a step of diafiltering the solution of step a) with a buffer solution, wherein the buffering agent is optionally a buffering agent comprising an amino acid, optionally histidine, in particular L-histidine Thus, during the diafiltration of the starting material a buffer exchange may be performed. It is noted that any definitions given herein for buffering agents comprising histidine also relate to buffering agents comprising an amino acid.

Examplary buffering agents comprising histidine include histidine, specifically L-histidine, histidine chloride, histidine hydrochloride monohydrate, histidine acetate, histidine phosphate, histidine sulfate and mixtures thereof. Particularly, the buffering agent comprising histidine is a buffering agent comprising histidine and/or histidine hydrochlorid monohydrate. It is understood, that any specification mentioned herein for histidine also relates to the exemplary histidine buffering agents mentioned above. As used herein a "buffering agent comprising histidine" relates to a pharmaceutically acceptable buffering agent comprising the amino acid histidine which suitable to keep the pH at a nearly constant value.

Particularly, the buffering agent is a buffering substance suitable to provide a pH value of 5.45 ±1.0. More particularly, the buffering substance is a buffering substance suitable to provide a pH value of 5.45±0.5. Specifically, the buffering substance is a buffering substance suitable to provide a pH value of 5.45±0.1. More specifically, the buffering substance is a buffering substance suitable to provide a pH value of 5.45±0.05.

In one embodiment of the invention, the buffer solution comprising histidine as a buffering agent comprises 30-60 mM histidine, particularly 35-45 mM histidine. In another embodiment of the invention, the buffer solution comprising histidine as a buffering agent comprises 30 mM histidine, 35 mM histidine, 40 mM histidine, 45 mM histidine, 50 mM histidine, 55 mM histidine or 60 mM histidine, particularly a buffer solution comprising 40 mM histidine.

In a specific embodiment of the invention, the buffer solution used for diafiltration is a buffer solution comprising 30-60 mM histidine, particularly 35-45 mM histidine and having a pH value of 5.45±2.0. In another specific embodiment of the invention, the buffer solution used for diafiltration is a buffer solution comprising 30-60 mM histidine, particularly 35-45 mM histidine and having a pH value of 5.45±1.0. In another specific embodiment of the invention, the buffer solution used for diafiltration is a buffer solution comprising 30-60 mM histidine, particularly 35-45 mM histidine and having a pH value of 5.45±0.5. In another specific embodiment of the invention, the buffer solution used for diafiltration is a buffer solution comprising 30-60 mM histidine, particularly 35-45 mM histidine and having a pH value of 5.45±0.1. In another specific embodiment of the invention, the buffer solution used for diafiltration is a buffer solution comprising 30-60 mM histidine, particularly 35-45 mM histidine and having a pH value of 5.45±0.05.

In a specific embodiment of the invention, the buffer solution used for diafiltration is a buffer solution comprising 40 mM histidine and having a pH value of 5.45±2.0. In another specific embodiment of the invention, the buffer solution used for diafiltration is a buffer solution comprising 40 mM histidine and having a pH value of 5.45±1.0. In another specific embodiment of the invention, the buffer solution used for diafiltration is a buffer comprising 40 mM histidine and having a pH value of 5.45±0.5. In another specific embodiment of the invention, the buffer solution used for diafiltration is a buffer comprising 40 mM histidine and having a pH value of 5.45±0.1. In another specific embodiment of the invention, the buffer solution used for diafiltration is a buffer solution comprising 40 mM histidine and having a pH value of 5.45±0.05.

If a buffer exchange is performed, it can be performed with at least 1-fold volume of the buffer solution compared to the antibody solution, with at least 2-fold volume of the buffer solution compared to the antibody solution, with at least 3-fold volume of the buffer solution compared to the antibody solution with at least 4-fold volume of the buffer solution compared to the antibody solution, with at least 5-fold volume of the buffer solution compared to the antibody solution, with at least 6-fold volume of the buffer solution compared to the antibody solution, with at least 7-fold volume of the buffer solution compared to the antibody solution, or with at least 8-fold volume of the buffer solution compared to the antibody solution. Particularly, the buffer exchange is performed with at least 8-fold volume of the buffer solution compared to the antibody solution.

In step b) of the method for preparation of a HCLF according to the invention the antibody or the fragment thereof is concentrated via ultrafiltration (UF) in order to obtain a solution having an intermediate concentration C^{I} of the antibody or a fragment thereof, wherein C^{I} is at least about 260 mg/mL. In one embodiment of the invention, the solution which is subjected to ultrafiltering in step b) contains about 40 mM histidine and/or has a pH value of 5.45±1.0. In another embodiment of the invention, the solution which is subjected to ultrafiltering in step b) contains about 40 mM histidine and/or has a pH value of 5.45±0.5. In a further embodiment of the invention, the solution which is subjected to ultrafiltering in step b) contains about 40 mM histidine and/or has a pH value of 5.45±0.1.

In a further embodiment, the solution which is subjected to ultrafiltering in step b) is essentially free of or does not contain a tonicity modifying agent as defined herein. In particular, the soltution subjected to ultrafiltering in step b) is essentially free of or does not contain sucrose.

In a further embodiment, the solution which is subjected to ultrafiltering in step b) is essentially free of or does not contain a surfactant as defined herein. In particular, the solution subjected to ultrafiltering in step b) is essentially free of or does not contain a non-ionic surfactant. More particularly, the solution which is subjected to ultrafiltering in step b) is essentially free of or does not contain polysorbate, wherein the polysorbate optionally is polysorbate 20.

Thus, according to one embodiment of the invention, solutions containing the antibody, optionally an anti-CD 20 antibody, particularly veltuzumab and a buffering agent comprising an amino acid, optionally histidine, are essentially free of or do not contain tonicity modifying agents and surfactants. In a specific embodiment, the solution subjected to ultrafiltration in step b), is an aqueous solution (i.e. an solution wherein the solvent is water) consisting of histidine and the antibody as defined herein.

As used herein "ultrafiltration" may denote any membrane filtration process for purifying and/or concentrating macromolecular solutions, wherein hydrostatic pressure is used in order to filtrate a liquid through a semipermeable membrane with appropriate chemical and physical properties. Such an ultrafiltration membrane is a membrane having a pore size between 0.01 and 0.1 µm and is used to separate particles having a size of about 0.1-0.01 µm, e.g. emulsions, proteins and macromolecules from suspension. The antibody as described herein is concentrated up to an intermediate concentration C^{I} which is higher than C^{H} and at least about 260 mg/mL. Typically, C^{I} is in the range of about 260-290 mg/mL, particularly about 270-290 mg/mL, more particularly about 270-285 mg/mL. In one embodiment of the invention, the antibody as described herein is concentrated up to a concentration C^{I} of at least 260 mg/mL. In some embodiments of the invention the antibody as described herein is concentrated up to a concentration C^{I} at least 265 mg/mL, at least 270 mg/mL, at least 275 mg/mL, at least 280 mg/mL, at least 285 mg/mL, at least 290 mg/mL, particularly up to a concentration C^{I} of at least 285 mg/mL. As already discussed above the term "about 280 mg/mL" relates to slight variations in a numerical value (i.e. 280 mg/mL) of ±10%, optionally ±5%. In one embodiment, the antibody is concentrated up to an intermediate concentration of 280 mg/mL.

In one embodiment of the method for preparation of a HCLF of an antibody or a fragment thereof, step b) is performed in an ultrafiltration device and the method further comprises between step b) and step c) a step wherein the device in which the ultrafiltration has been performed is flushed with a buffer solution, optionally a buffer solution comprising an amino acid as defined herein. Particularly, the buffering agent in the buffer solution used for flushing the ultrafiltration device is histidine, specifically L-histidine. In addition or alternatively, the buffering agent used for flushing is identical with the buffering agent contained in the solution of step a). This flushing step may allow further minimizing the loss of antibodies when the highly concentrated antibody composition is removed from the ultrafiltation device, thus further maximizing the yield of the method described herein.

In a further step c) of the method described herein the highly concentrated antibody composition obtained in step b) may be further diluted to obtain the HCLF in the desired final concentration.

In step c) the HCLF in the desired final concentration of the antibody or fragment thereof may be obtained by any method known in the art. The method chosen as well as the components used in order to obtain the desired final concentration of the antibody or fragment thereof may depend on the desired final composition of the HCLF.

In one of its embodiments step c) of the method according to the invention thus comprises diluting the highly concentrated antibody composition obtained in step c) with a buffer solution.

This buffer solution may comprise any of the aforementioned buffering agents such as buffering agents comprising amino acids, in particular comprising histidine. In Particular, the buffer solution comprises 30-50 mM histidine, optionally 30 mM histidine.

Furthermore, the pH value of the buffer solution used for diluting the highly concentrated antibody composition obtained from step b) may be in the range of 4.8 to 7.0, in particular in the range of 5 to 6. Optionally, the pH value of said buffer solution is 5.5±2.0, 5.5±1.0, 5.5±0.5, 5.5±0.3 or 5.5±0.2. In one embodiment, the pH value of said buffer solution is 5.5±0.3. In another embodiment, the pH value of said buffer solution is 5.5±0.2.

In particular, this buffer solution may be a buffer solution further comprising a surfactant as defined herein. In one embodiment of the method according to the invention, the buffer solution comprising a surfactant is a buffer solution comprising a non-ionic surfactant, in particular a polysorbate. In a specific embodiment of the method according to the invention, the buffer solution comprising a surfactant, is a buffer solution comprising polysorbate 20 or polysorbate 80, particularly polysorbate 20.

The buffer solution may comprise the 5-fold, the 6-fold, the 7-fold, the 8-fold, the 9-fold or 10-fold concentration of the desired concentration of surfactant in the final product. In a specific embodiment, the buffer solution has the 10-fold concentration of the desired concentration of surfactant in the final product.

In particular, the buffer solution comprises a concentration of surfactant of at least 0.1 g/L, at least 0.2 g/L, at least 0.3 g/L, at least 0.4 g/L, at least 0.5 g/L, at least 0.6 g/L, at least 0.7 g/L, at least 0.8 g/L, at least 0.9 g/L, at least 1 g/L, at least 2 g/L, at least 3 g/L, at least 4 g/L, at least 5 g/L, at least 6 g/L, at least 7 g/L, at least 8 g/L, at least 9 g/L or at least 10 g/L.

In one embodiment, the concentration of surfactant in the buffer solution is in the range of 0.1-1 g/L, in particular in the range of 0.3 g/L to 1 g/L. In this embodiment the surfactant is in particular polysorbate 80. In another embodiment the concentration of surfactant in the buffer solution is in the range of 1-10 g/L, in particular in the range of 1-3 g/L. In this embodiment the surfactant is in particular polysorbate 20.

In a specific embodiment the buffer solution comprises 0.2 g/L surfactant, particularly 0.2 g/L polysorbate 80. In another specific embodiment the buffer solution comprises 2 g/L surfactant, particularly 2 g/L polysorbate 20.

The buffer solution may further comprise a tonicity modifying agent. The tonicity modifying agent may be selected from NaCl, potassium chloride, glycine, glycerol, salts, amino acids and sugars. Optionally, the tonicity modifying agent is a sugar or sugar alcohol, e.g. selected from sucrose, trehalose, mannitol, sorbitol and glucose. In particular, the tonicity modifying agent is selected from sorbitol, sucrose and/or mannitol. In one embodiment, the tonicity modifying agent is sorbitol. In one embodiment, the tonicity modifying agent is mannitol. In one embodiment, the tonicity modifying agent is sucrose.

The buffer solution may comprise the 5-fold, the 6-fold, the 7-fold, the 8-fold, the 9-fold or the 10-fold concentration of the desired concentration of tonicity modifying agent in the final product. In particular, the buffer solution comprises a 10-fold concentration of tonicity modifying agent of the desired concentration of tonicity modifying agent in the final product.

In particular, the buffer solution may comprise at least 1 M, at least 1.5 M, at least 2 M, at least 2.2 M, at least 2.4 M, at least 2.6 M, at least 2.8 M, at least 3 M tonicity modifying agent. Optionally, the concentration of tonicity modifying agent in the buffer solution of the present invention is in the range of 0.05 M-5.5 M, in particular 1-3 M, more particularly 2-2.5 M. In a specific embodiment of the invention, the concentration of the tonicity modifying agent is 2.2 M.

The solution derived from said dilution step c) may have a concentration of the antibody or fragment thereof of at least 50 mg/mL, at least 70 mg/mL, at least 75 mg/mL, at least 80 mg/mL, at least 90 mg/mL, at least 100 mg/mL, at least 110 mg/mL, at least 120 mg/mL, at least 130 mg/mL, at least 140 mg/mL, at least 150 mg/mL, at least 155 mg/mL, at least 160 mg/mL, at least 170 mg/ml, at least 175 mg/mL, at least 180 mg/mL, at least 190 mg/mL, or at least 200 mg/mL, in particular of at least 175 mg/mL. In one embodiment, the composition obtained in said dilution step has a concentration of the antibody or fragment thereof of 100-290 mg/mL, 130-200 mg/mL, 155-200 mg/mL, 160-200 mg/mL or 175-200 mg/mL.

The concentration of the surfactant in the composition derived from said dilution step may be any concentration considered suitable by the person skilled in the art. In particular, the composition may comprise a concentration of surfactant of at least 0.01 g/L, at least 0.02 g/L, at least 0.03 g/L, at least 0.04 g/L, at least 0.05 g/L, at least 0.06 g/L, at least 0.07 g/L, at least 0.08 g/L, at least 0.09 g/L, at least 0.1 g/L, at least 0.2 g/L, at least 0.3 g/L, at least 0.4 g/L, at least 0.5 g/L, at least 0.6 g/L, at least 0.7 g/L, at least 0.8 g/L, at least 0.9 g/L or at least 1.0 g/L surfactant in the antibody composition after dilution has been performed. In one embodiment, the concentration of surfactant in the composition after dilution is in the range of 0.01-0.1 g/L, in particular 0.03 g/L to 0.1 g/L. In this embodiment the surfactant is in particular polysorbate 80.

In another embodiment the concentration of surfactant in the composition after dilution is in the range of 0.1-1.0 g/L, in particular 0.1-0.3 g/L. In this embodiment the surfactant is in particular polysorbate 20.

In a specific embodiment the concentration of surfactant in the composition after dilution is 0.02 g/L surfactant, particularly 0.02 g/L polysorbate 80. In another specific embodiment the concentration of surfactant in the composition after dilution is 0.2 g/L surfactant, particularly 0.2 g/L polysorbate 20.

The concentration of the tonicity modifying agent in the composition derived from the dilution step may be any concentration considered suitable by the person skilled in the art. In particular, the composition may comprise a concentration of tonicity modifying agent of at least 100 mM, at least 150 mM, at least 200 mM, at least 220 mM, at least 240 mM, at least 260 mM, at least 280 mM, at least 300 mM. Optionally, the concentration of tonicity modifying agent in the antibody composition after dilution is in the range of 100-300 mM, in particular 200-250 mM.

Step c) of the method according to the invention may include a second dilution step for final adjustment of the concentration of the antibody or fragment thereof in the HCLF. In particular, this step includes dilution with a so-called formulation buffer solution.

The formulation buffer solution used in step c) may comprise a surfactant as defined herein. In one embodiment, the surfactant is a non-ionic surfactant, in particular a polysorbate. In a specific embodiment, the surfactant is polysorbate 20 or polysorbate 80, particularly polysorbate 20. The concentration of the surfactant in the HCLF obtained according to the method of to the present invention is a concentration of at least 0.01 g/L, at least 0.02 g/L, at least 0.03 g/L, at least 0.04 g/L, at least 0.05 g/L, at least 0.06 g/L, at least 0.07 g/L, at least 0.08 g/L, at least 0.09 g/L, at least 0.1 g/L, at least 0.2 g/L, at least 0.3 g/L, at least 0.4 g/L, at least 0.5 g/L, at least 0.6 g/L, at least 0.7 g/L, at least 0.8 g/L, at least 0.9 g/L or at least 1.0 g/L surfactant.

In one embodiment the concentration of the surfactant in the formulation buffer solution of to the present invention is in the range of 0.01-0.1 g/L, in particular 0.03 g/L to 0.1 g/L. In this embodiment the surfactant is in particular polysorbate 80.

In another embodiment the concentration of surfactant in the formulation buffer solution is in the range of 0.1-1.0 g/L, in particular 0.1-0.3 g/L. In this embodiment the surfactant is in particular polysorbate 20.

The formulation buffer solution used in the method of the present invention may further comprise a tonicity modifying agent. The tonicity modifying agent may be selected from NaCl, potassium chloride, glycine, glycerol, salts, amino acids, sugar alcohols and sugars. Optionally, the tonicity modifying agent is a sugar or sugar alcohol, e.g. selected from glucose, sucrose, trehalose, mannitol, sorbitol and glucose. In particular, the tonicity modifying agent is sorbitol and/or mannitol. In one embodiment, the tonicity modifying agent is sorbitol. The concentration of the tonicity modifying agent in said buffer solution may be any concentration considered suitable by the person skilled in the art. In particular, the formulation buffer solution used in the method of the present invention may comprise at least 100 mM, at least 150 mM, at least 200 mM, at least 220 mM, at least 240 mM, at least 260 mM, at least 280 mM, at least 300 mM tonicity modifying agent. Optionally, the concentration of tonicity modifying agent in the formulation buffer solution used in the method of the present invention is in the range of 100-300 mM, in particular 200-250 mM. In a specific embodiment the formulation buffer solution comprises 220 mM tonicity modifying agent, in particular 220 mM sorbitol.

The formulation buffer solution further comprises a buffering agent as defined herein. In particular, the buffering agent is a buffering agent comprising an amino acid, optionally comprising histidine, specifically L-histidine, histidine chloride, histidine hydrochloride monohydrate, histidine acetate, histidine phosphate, or histidine sulfate or mixtures thereof. Particularly, the formulation buffer solution comprises histidine and/or histidine hydrochlorid monohydrate.

In one embodiment of the invention, the formulation buffer solution comprises 30-60 mM histidine, particularly 35-45 mM histidine. In another embodiment of the invention, the formulation buffer solution comprises about 30 mM histidine, about 35 mM histidine, about 40 mM histidine, about 45 mM histidine, about 50 mM histidine, about 55 mM histidine or about 60 mM histidine, particularly the formulation buffer solution comprises 30 mM histidine. In another embodiment, the formulation buffer solution comprises at least 50 mM histidine.

Furthermore, the pH value of the formulation buffer solution used in step c) may be in the range of 4.8 to 7.0, in particular in the range of 5 to 6. Optionally, the pH value of said buffer is 5.5±2.0, 5.5±1.0, 5.5±0.5, 5.5±0.3 or 5.5±0.2. In one embodiment, the pH value of said buffer is 5.5±0.3. In another embodiment, the pH value of said buffer is 5.5±0.2.

The method as described herein is performed at room temperature, a definition of said term is provided above. Specifically, all method steps as described herein are performed at 18 °C to 25 °C, optionally at 19 °C to 22 °C.

The HCLF obtained according to the method of the present invention has an antibody concentration or concentration of a fragment thereof as defined above for HCLFs.

The pH value of the HCLF obtained according to the method of to the present invention may be in the range of 4.8 to 7.0, in particular in the range of 5.0 to 6.0. In one embodiment the HCLF obtained according to the method of to the present invention has a pH of 5.5±2.0, 5.5±1.0, 5.5±0.5, 5.5±0.3 or 5.5±0.2. In a specific embodiment, the pH value of the liquid pharmaceutical formulation of the present invention is 5.5±0.3. In another specific embodiment, the pH value of the liquid pharmaceutical formulation of the present invention is 5.5±0.2.

The HCLF obtained according to the method of to the present invention comprises a buffering agent as defined herein. In particular, the buffering agent is a buffering agent comprising an amino acid, optionally a buffering agent comprising histidine, specifically L-histidine, histidine chloride, histidine hydrochloride monohydrate, histidine acetate, histidine phosphate, or histidine sulfate or mixtures thereof. Particularly, the HCLF obtained according to the method of to the present invention comprises histidine and/or histidine hydrochlorid monohydrate.

In one embodiment of the invention, the HCLF obtained according to the method of to the present invention comprises 30-60 mM histidine, particularly 35-45 mM histidine. In another embodiment of the invention, the HCLF obtained according to the method of to the present invention comprises about 30 mM histidine, about 35 mM histidine, about 40 mM histidine, about 45 mM histidine, about 50 mM histidine, about 55 mM histidine or about 60 mM histidine, particularly the HCLF obtained according to the method of to the present invention comprises 30 mM histidine. In another embodiment, the HCLF comprises at least 50 mM histidine.

The HCLF obtained according to the method of to the present invention may comprise a surfactant as defined herein. In one embodiment, the surfactant is a non-ionic surfactant, in particular a polysorbate. In a specific embodiment, the surfactant is polysorbate 20 or polysorbate 80, particularly polysorbate 20. The concentration of the surfactant in the HCLF obtained according to the method of to the present invention is a concentration of at least 0.01 g/L, at least 0.02 g/L, at least 0.03 g/L, at least 0.04 g/L, at least 0.05 g/L, at least 0.06 g/L, at least 0.07 g/L, at least 0.08 g/L, at least 0.09 g/L, at least 0.1 g/L, at least 0.2 g/L, at least 0.3 g/L, at least 0.4 g/L, at least 0.5 g/L, at least 0.6 g/L, at least 0.7 g/L, at least 0.8 g/L, at least 0.9 g/L or at least 1.0 g/L surfactant.

In one embodiment, the concentration of the surfactant in the HCLF obtained according to the method of to the present invention is in the range of 0.01-0.1 g/L, in particular 0.03 g/L to 0.1 g/L. In this embodiment the surfactant is in particular polysorbate 80.

In another embodiment the concentration of surfactant in the composition after dilution is in the range of 0.1-1.0 g/L, in particular 0.1-0.3 g/L. In this embodiment the surfactant is in particular polysorbate 20.

The HCLF obtained according to the method of the present invention may further comprise a tonicity modifying agent. The tonicity modifying agent may be selected from NaCl, potassium chloride, glycine, glycerol, salts, amino acids, sugar alcohols and sugars. Optionally, the tonicity modifying agent is a sugar or sugar alcohol, e.g. selected from glucose, sucrose, trehalose, mannitol, sorbitol and glucose. In particular, the tonicity modifying agent is sorbitol and/or mannitol. In one embodiment, the tonicity modifying agent is sorbitol. The concentration of the tonicity modifying agent in said buffer may be any concentration considered suitable by the person skilled in the art. In particular, the HCLF obtained according to the method of the present invention may comprise at least 100 mM, at least 150 mM, at least 200 mM, at least 220 mM, at least 240 mM, at least 260 mM, at least 280 mM, at least 300 mM tonicity modifying agent. Optionally, the concentration of tonicity modifying agent in the HCLF obtained according to the method of the present invention is in the range of 5.5 mM-550 mM, in particular 100-300 mM, more particularly 200-250 mM. In a specific embodiment the concentration of the tonicity modifying agent in the HCLF obtained according to the method of the present invention is 220 mM. In another embodiment of the invention, the concentration of the tonicity modifying agent of the present invention is at least 270 mM.

It is understood that the HCLF obtained according to the method of to the present invention may further comprise any further pharmaceutically acceptable compound considered useful by the person skilled in the art. Examples of further pharmaceutically acceptable compounds include pharmaceutically acceptable excipients, additives, diluents, chelating agents, lyoprotectants, adjuvants, delivery vehicles and anti-microbial preservatives which may be added in order to provide a desired property to the final formulation. Such additional compounds are known to the person skilled in the art and may be chosen according to the desired property. They may be added at any step during the above process considered suitable by the person skilled in the art. In particular, the further pharmaceutically acceptable compounds may be added during the first dilution step of step c).

In one embodiment the HCLF obtained according to the method of the present invention further comprises arginine in a concentration considered suitable by the person skilled in the art. The addition of arginine allows for a further stabilization of the antibody and an increased melting point.

Exemplary embodiments of the method for providing a HCLF according to the invention are shown in figures 1 to 3 and described in detail in the figure description.

During storage, antibody formulations can undergo chemical and/or physical degradation, thus leading to unwanted degradation products such as aggregates. This degradation may lead to a reduced potency of the antibody and increase the risk of unwanted side effects (e.g. unwanted immune responses) which may lead to a significant impact on regulatory approval of the drug. In particular, at higher concentrations of the antibody, the tendency to form aggregates may increase dramatically. However, it has now been found that an anti-CD 20 antibody or fragment thereof may be stabilized and solubilized in a liquid pharmaceutical formulation at high concentrations by using an aqueous solution comprising an amino acid. Amino acids include any aminoc acid considered suitable by the person skilled in the art such as alanine, arginine, asparagine, aspartic acid, cysteine, glutamic acid, glutamin, glycine, histidine, isoleucine, leucine, lysine, methionine, phenylalanine, proline, serine, threonine, tryptophan, tyrosine, and valine. In particular, the amino acid should act as a buffering agent. In one embodiment of the invention, the aqueous solution comprises histidine, optionally L-histidine. It is noted that any specification provided herein for histidine is also applicable for L-histidine.

In a further aspect, the present invention thus relates to a method for stabilizing an anti-CD 20 antibody or a fragment thereof in a liquid pharmaceutical formulation in a concentration of at least 155 mg/mL by combining the antibody or fragment thereof, without subjecting it to prior lyophilization or freeze drying, with an aqueous solution comprising an amino acid, optionally histidine.

In one of its embodiments, the liquid pharmaceutical formulation comprises at least 160 mg/mL, at least 175 mg/mL, at least 180 mg/mL, at least 185 mg/mL, at least 190 mg/mL, at least 195 mg/mL, at least 200 mg/mL, at least 220 mg/mL, at least 240 mg/mL, at least 260 mg/mL, at least 280 mg/mL, at least 285 mg/mL, or at least 290 mg/mL of a veltuzumab antibody or a fragment thereof. In one specific embodiment, the liquid pharmaceutical formulation comprises at least 160 mg/mL of a veltuzumab antibody or a fragment thereof. In another specific embodiment, the liquid pharmaceutical formulation comprises at least 175 mg/mL of a veltuzumab antibody or a fragment thereof. In a further specific embodiment, the liquid pharmaceutical formulation comprises at least 200 mg/mL of a veltuzumab antibody or a fragment thereof. In another embodiment of the invention, the liquid pharmaceutical formulation of the present invention comprises 160-290 mg/mL, 160-250 mg/mL, 160-220 mg/mL, 160-200 mg/mL, or 160-175 mg/mL of a veltuzumab antibody or a fragment thereof.

According to the method of the present invention, the antibody or fragment thereof has not been subject to prior lyophilization or freeze-drying. "Lyophilization" as used herein relates to any dehydration process wherein a formulation is freezed and subsequently the surrounding pressure is reduced in order to allow for sublimation of the frozen water from the freezed formulation. Such a process may be performed by any lyophilization method known in the art.

In the method according to the invention the antibody or fragment thereof is combined with an aqueous solution comprising an amino acid, optionally histidine. As used herein, aqueous solution relates to any solution in which the solvent is water.

The aqueous solution comprising histidine may comprise only histidine as buffering agent, histidine chloride, histidine hydrochloride monohydrate, histidine acetate, histidine phosphate, or histidine sulfate or combinations thereof. Particularly, the aqueous solution comprising histidine used in the method of the present invention comprises only histidine as buffering agent or histidine in combination with histidine hydrochlorid monohydrate as buffering agent.

The aqueous solution comprising an amino acid, optionally histidine may comprise at least one further therapeutically acceptable buffering agent in addition to the amino acid, optionally in addition to histidine or in addition to the combination of histidine containing buffering agents mentioned above. Such further buffering agents include phosphate, acetate (e.g., sodium acetate), succinate (such as sodium succinate), gluconate, glutamate, histidine, citrate, other amino acids mentioned herein acting as buffering agents and other organic acid buffers.

The pH value of the aqueous solution comprising an amino acid, optionally histidine may be in the range of 4.8 to 7.0, in particular in the range of 5.0 to 6.0. In one embodiment the pH value of the aqueous solution comprising an amino acid, optionally histidine can be 5.5±2.0, 5.5±1.0, 5.5±0.5, 5.5±0.3 or 5.5±0.2. In a specific embodiment, the pH value of the aqueous solution comprising an amino acid, optionally histidine used in the method of the present invention is 5.5±0.3. In another specific embodiment, the pH value of the aqueous solution comprising an amino acid, optionally histidine used in method of the present invention is 5.5±0.2.

In one embodiment of the invention, the aqueous solution comprising an amino acid, optionally histidine used in the method of to the present invention comprises 30-60 mM amino acid, optionally histidine, particularly 30-45 mM amino acid, optionally histidine. In another embodiment of the invention, the aqueous solution comprising amino acid, optionally histidine used in method of to the present invention comprises 30 mM amino acid, optionally histidine, 35 mM amino acid, optionally histidine, 40 mM amino acid, optionally histidine, 45 mM amino acid, optionally histidine, 50 amino acid, optionally mM histidine, 55 mM amino acid, optionally histidine or 60 mM amino acid, optionally histidine. Particularly, the aqueous solution comprising an amino acid, optionally histidine used in the method of to the present invention comprises 30 mM amino acid, optionally histidine. In another embodiment, the aqueous solution comprising an amino acid, optionally histidine comprises at least 50 mM amino acid, optionally histidine.

The aqueous solution comprising an amino acid, optionally histidine used in the method of the present invention may further comprise a tonicity modifying agent.

The tonicity modifying agent may be selected from NaCl, potassium chloride, glycine, glycerol, salts, amino acids, sugar alcohols and sugars. Optionally, the tonicity modifying agent is a sugar or sugar alcohol, e.g. selected from glucose, sucrose, trehalose, mannitol, sorbitol and glucose. In particular, the tonicity modifying agent is sorbitol, sucrose and/or mannitol. In one embodiment, the tonicity modifying agent is sorbitol. In another embodiment, the tonicity modifying agent is sucrose. In a further embodiment, the tonicity modifying agent is mannitol. The concentration of the tonicity modifying agent in said buffer may be any concentration considered suitable by the person skilled in the art. The aqueous solution comprising an amino acid, optionally histidine used in the method of the present invention may comprise at least 4%, at least 5%, at least 6%, at least 7%, at least 8%, at least 9% or at least 10% tonicity modifying agent. In one embodiment of the invention, the aqueous solution comprising an amino acid, optionally histidine comprises from 5-8% tonicity modifying agent.

Addition of a surfactant leads to a further reduction of the formation of degradation products, in particular a reduced formation of aggregates, thereby helping to stabilize the antibody formulation. Thus, the aequous solution comprising an amino acid, optionally histidine used in the method of to the present invention may comprise a surfactant as defined herein. In one embodiment, the surfactant is a non-ionic surfactant, in particular a polysorbate. In a specific embodiment, the surfactant is polysorbate 20 or polysorbate 80, particularly polysorbate 20. The aqueous solution comprising amino acid, optionally histidine used in the method according to the invention may comprise any amount of surfactant considered useful by the person skilled in the art, in particular at least 0.01%, at least 0.02%, at least 0.03%, at least 0.04%, at least 0.05%, at least 0.1% or at least 1.0 % surfactant. Optionally, the aequous solution comprising an amino acid, optionally histidine used in the method according to the present invention comprises at least 0.01 % surfactant.

Examples of anti-CD 20 antibodies which may be stabilized according to the above described method are Veltuzumab, Rituximab, Ocrelizumab, Ofatumumab, Y⁹⁰.Ibritumomab tiuxetan, I¹³¹ tositumab, TRU-015, AME-133v, PRO131921 humanized, GA101, 1F5 IgG2a, HI47 IgG3, 2C6 IgG1, 2H7 IgG1, AT80 IgG1, 11B8 IgG1, humanized B-Ly1 antibody IgG1 and Aftuzumab (HuMab<CD20>) or fragments thereof. Particularly, the anti-CD20 antibody stabilized with the above described method is veltuzumab or a fragment thereof.

As used herein, "stable" or "stabilized" in relation to an antibody formulation of the present invention relates to an antibody formulation retaining its chemical stability and/or physical stability during storage. Methods for determining the stability of an antibody formulation are known in the art. In particular, a stable/stabilized antibody formulation retains its chemical and/or physical stability upon storage at room temperature for at least one month, optionally for at least three months upon storage at 25 °C and/or upon storage at 2-8°C for at least one year.

In one specific embodiment, the liquid formulation of the anti-CD20 antibodies or fragments thereof provided by any of the above described methods comprises
a) at least 160 mg/mL anti-CD20 antibody or a fragment thereof, optionally a veltuzumab antibody or a fragment thereof;
b) 220 mM sorbitol;
c) 30 mM histidine;
d) 0.2 g/L polysorbate 20; and
and has a pH value in the range of 4.8 to 7.0, in particular in the range of 5.0 to 6.0. In a specific embodiment the above liquid formulation has a pH value of 5.5±0.3. In another specific embodiment, the above liquid formulation has a pH value of 5.5±0.2.

The liquid formulations of the anti-CD20 antibodies or fragments thereof provided by the above described methods are suitable for long-term storage and may also be lyophilized in order to even enhance the term of storage.

An exemplary method for stabilizing an anti-CD 20 antibody is shown in the examples below.

It has further been found that a liquid pharmaceutical formulation comprising at least 155 mg/mL of a veltuzumab antibody or a fragment thereof with reduced tendency to form aggregates may be provided.

In one aspect the present invention thus relates to a liquid pharmaceutical formulation of a veltuzumab antibody or a fragment thereof comprising at least 155 mg/mL veltuzumab antibody or a fragment thereof and an amino acid. Amino acids include any amino acid considered suitable by the person skilled in the art such as alanine, arginine, asparagine, aspartic acid, cysteine, glutamic acid, glutamin, glycine, histidine, isoleucine, leucine, lysine, methionine, phenylalanine, proline, serine, threonine, tryptophan, tyrosine, and valine In particular, the amino acid should act as a buffering agent. In one embodiment of the invention, the liquid pharmaceutical formulation comprises histidine, optionally L-histidine. It is noted that any specification provided herein for histidine is also applicable for L-histidine.

In one of its embodiments, the liquid pharmaceutical formulation comprises at least 160 mg/mL, at least 175 mg/mL, at least 180 mg/mL, at least 185 mg/mL, at least 190 mg/mL, at least 195 mg/mL, at least 200 mg/mL, at least 220 mg/mL, at least 240 mg/mL, at least 260 mg/mL, at least 280 mg/mL, at least 285 mg/mL, or at least 290 mg/mL of a veltuzumab antibody or a fragment thereof. In one specific embodiment, the liquid pharmaceutical formulation comprises at least 160 mg/mL of a veltuzumab antibody or a fragment thereof. In another specific embodiment, the liquid pharmaceutical formulation comprises at least 175 mg/mL of a veltuzumab antibody or a fragment thereof. In a further specific embodiment, the liquid pharmaceutical formulation comprises at least 200 mg/mL of a veltuzumab antibody or a fragment thereof. In another embodiment of the invention, the liquid pharmaceutical formulation of the present invention comprises 160-290 mg/mL, 160-250 mg/mL, 160-220 mg/mL, 160-200 mg/mL, or 160-175 mg/mL of a veltuzumab antibody or a fragment thereof.

The liquid pharmaceutical formulation of the present invention comprises an amino acid, optionally histidine as a buffering agent. It may comprise only an amino acid, optionally histidine as buffering agent or additionally histidine, histidine chloride, histidine hydrochloride monohydrate, histidine acetate, histidine phosphate, or histidine sulfate or combinations thereof. Particularly, the liquid pharmaceutical formulation of the present invention comprises only histidine as buffering agent or histidine in combination with histidine hydrochloride monohydrate as buffering agent.

The liquid pharmaceutical formulation of the present invention may comprise at least one further therapeutically acceptable buffering agent in addition to the amino acid, optionally histidine or in addition to the combination of histidine containing buffering agents mentioned above. Such further buffering agents include phosphate, acetate (e.g., sodium acetate), succinate (such as sodium succinate), gluconate, glutamate, histidine, citrate, further amino acids and other organic acid buffers.

In one embodiment of the invention, the concentration of the buffering agent, optionally histidine in the liquid pharmaceutical formulation of the present invention is in the range of 1 mM to 100 mM. In another embodiment, the concentration of the buffering agent, optionally histidine in the liquid pharmaceutical formulation of the present invention is in the range of 10 mM to 60 mM or 10 mM to 50 mM, optionally in the range of 20 mM to 40 mM. The liquid pharmaceutical formulation of the present invention may comprise 10 mM of the buffering agent, optionally histidine, 15 mM of the buffering agent, optionally histidine, 20 mM of the buffering agent, optionally histidine, 25 mM of the buffering agent, optionally histidine, 30 mM of the buffering agent, optionally histidine, 35 mM of the buffering agent, optionally histidine, 40 mM of the buffering agent, optionally histidine, 45 mM of the buffering agent, optionally histidine, 50 mM of the buffering agent, optionally histidine, 55 mM of the buffering agent, optionally histidine or 60 mM of the buffering agent, optionally histidine, particularly it comprises 30 mM of the buffering agent, optionally histidine. In another embodiment, it comprises at least 50 mM of the buffering agent, optionally histidine.

The liquid pharmaceutical formulation of the present invention may further comprise a surfactant as defined herein. The addition of a surfactant may lead to a further reduction of the formation of degradation products, in particular a reduced formation of aggregates. In one embodiment, the surfactant is a non-ionic surfactant, in particular a polysorbate. In a specific embodiment, the surfactant is polysorbate 20 or polysorbate 80, particularly polysorbate 20. The liquid pharmaceutical formulation according to the invention may comprise any amount of surfactant considered useful by the person skilled in the art. The liquid pharmaceutical formulation according to the invention may comprise 0.01 to 1.0 g/L surfactant. In particular it may comprise at least 0.01 g/L, at least 0.02 g/L, at least 0.03 g/L, at least 0.04 g/L, at least 0.05 g/L, at least 0.06 g/L, at least 0.07 g/L, at least 0.08 g/L, at least 0.09 g/L, at least 0.01 g/L at least 0.02 g/L, at least 0.03 g/L, at least 0.04 g/L, at least 0.05 g/L, at least 0.1 g/L, at least 0.2 g/L or at least 1.0 g/L surfactant.

In one embodiment, the concentration of the surfactant in the liquid pharmaceutical formulation of to the present invention is in the range of 0.01-0.1 g/L, in particular 0.03 g/L-0.1 g/L. In this embodiment the surfactant is in particular polysorbate 80.

In another embodiment the concentration of surfactant in the liquid pharmaceutical formulation is in the range of 0.1-1.0 g/L, in particular 0.1-0.3 g/L. In this embodiment the surfactant is in particular polysorbate 20.

The liquid pharmaceutical formulation of the present invention may further comprise a tonicity modifying agent. The tonicity modifying agent may be selected from NaCl, potassium chloride, glycine, glycerol, salts, amino acids, sugar alcohols and sugars. Optionally, the tonicity modifying agent is a sugar or sugar alcohol, e.g. selected from glucose, sucrose, trehalose, mannitol, sorbitol and glucose. In particular, the tonicity modifying agent is sorbitol, sucrose and/or mannitol, more particularly sorbitol and/or mannitol. In one embodiment, the tonicity modifying agent is sorbitol. In another embodiment, the tonicity modifying agent is sucrose. In a further embodiment, the tonicity modifying agent is mannitol. The concentration of the tonicity modifying agent in said buffer may be any concentration considered suitable by the person skilled in the art. In one embodiment of the invention, the concentrations of the tonicity modifying agent in the liquid pharmaceutical formulation of the present invention is in the range from 5.5 mM-550 mM, in particular 100-300 mM, more particularly 200-250 mM. In a specific embodiment the concentration of the tonicity modifying agent in the buffer is 220 mM. In particular, the concentration of the tonicity modifying agent in the liquid pharmaceutical formulation of the present invention is at least 100 mM, at least 150 mM, at least 200 mM, at least 220 mM, at least 240 mM, at least 260 mM, at least 280 mM,or at least 300 mM tonicity modifying agent. In one embodiment of the invention, the concentration of the tonicity modifying agent of the present invention is at least 220 mM. In another embodiment of the invention, the concentration of the tonicity modifying agent of the present invention is at least 270 mM.

The pH value of the liquid pharmaceutical formulation of the present invention may be in the range of 4.8 to 7.0, in particular in the range of 5.0 to 6.0. In one embodiment of the invention, the pH value of the liquid pharmaceutical formulation of the present invention is 5.5±2.0, 5.5±1.0, 5.5±0.5, 5.5±0.3 or 5.5±0.2. In a specific embodiment, the pH value of the liquid pharmaceutical formulation of the present invention is 5.5±0.3. In another specific embodiment, the pH value of the liquid pharmaceutical formulation of the present invention is 5.5±0.2.

The pH value in the liquid pharmaceutical formulation of the present invention may be adjusted with any acid or base considered suitable by the person skilled in the art, in particular a pharmaceutically acceptable acid or base. Examples include hydrochloric acid, sodium hydroxide and acetic acid.

In one embodiment of the invention, the liquid pharmaceutical formulation according to the present invention comprises
a) at least 160 mg/mL veltuzumab antibody or a fragment thereof;
b) 220 mM sorbitol;
c) 30 mM histidine;
d) 0.2 mg/mL polysorbate 20; and
and has a pH value in the range of 4.8 to 7.0, in particular in the range of 5.0 to 6.0. In a specific embodiment the above liquid formulation has a pH value of 5.5±0.3. Optionally, the liquid pharmaceutical formulation has a pH value of 5.5±0.2.

The liquid pharmaceutical formulation of the present invention may further comprise additional components such as pharmaceutically acceptable excipients, additives, diluents, chelating agents, lyoprotectants, adjuvants, delivery vehicles and anti-microbial preservatives which may be added in order to provide a desired property to the final formulation. Such additional components are known to the person skilled in the art and may be chosen according to the desired property. In one embodiment the liquid pharmaceutical formulation of the present invention further comprises arginine for further stabilization and increasing the melting point in a concentration considered suitable by the person skilled in the art.

Another embodiment of the invention relates to the liquid pharmaceutical formulation as described herein for use as a medicament. It is understood herein, that also the HCLF derived by the above described method or the liquid pharmaceutical formulation stabilized by the above described method may also be used as a medicament and that explanation given below with regards to the liquid pharmaceutical formulation also relates to any of the formulation derived by the above described methods.

In one embodiment, the liquid pharmaceutical formulation or the HCLF as described herein may be used in the treatment of cancer or a non-malignant disease, optionally inflammatory or autoimmune diseases.

"Cancer" as used herein may relate to any malignant disease involving unregulated cell growth, e.g. Epstein-Barr Virus Infections, Leukemia, Lymphoma, Plasma Cell Neoplasms, Tumor Virus Infections, Immunoproliferative Disorders, Lymphoproliferative Disorders, Paraproteinemias, Herpesviridae Infections, DNA Virus Infections.

In particular, the liquid pharmaceutical formulation or the HCLF as described herein are useful for the treatment of any CD 20 positive cancers, i.e. cancers showing abnormal proliferation of cells that express CD 20 on the cell surface, in particular T-cells or B-cells. Methods for determining the expression of CD 20 on the cell surface are known in the art and include e.g. FACS or PCR. Exemplary CD 20 positive cancers which can be treated with the liquid pharmaceutical formulation or the HCLF according to the invention are B cell lymphomas and leukemias.

Lymphomas and leukemias include Burkitt Lymphoma, B-Cell Leukemia, Chronic Lymphocytic B-Cell Leukemia, Acute Lymphoblastic Leukemia, Lymphoid Leukemia, Prolymphocytic Leukemia, Hairy Cell Leukemia, Multiple Myeloma, posttransplant lymphoproliverative disorder (PTLD), HIV-associated Lymphoma, Primary CNS Lymphoma, B-Cell Lymphoma, Marginal Zone B-Cell Lymphoma, Follicular Lymphoma, Diffuse Large B-Cell Lymphoma, Immunoblastic Large-Cell Lymphoma, Mantle-Cell Lymphoma, Non-Hodgkin Lymphoma (e.g. Follicular Lymphoma), Lymphomatoid Granulomatosis, Precursor Cell Lymphoblastic Leukemia-Lymphoma, Waldenstrom Macroglobulinemia, Prolymphocytic Lymphoma, Diffuse Large B-Cell Lymphoma, Immunoblastic Large-Cell Lymphoma, Mantle-Cell Lymphoma, Lymphomatoid Granulomatosis and Precursor Cell Lymphoblastic Leukemia-Lymphoma. Optionally, the CD 20 positive cancer is a disease selected from the group consisting ofB-cell non-Hodgkin's Lymphoma, Mantle-Cell Lymphoma, Acute Lymphoblastic Leukemia, Chronic Lymphocytic B-Cell Leukemia, Diffuse Large B-Cell Lymphoma, Burkitt Lymphoma, Follicular Lymphoma, Multiple Myeloma, Marginal Zone B-Cell Lymphoma, posttransplant lymphoproliverative disorder (PTLD), HIV-associated Lymphoma, Waldenstrom Macroglobulinemia, or Waldenstrom Macroglobulinemia. Particularly, the CD 20 positive cancer is a B-cell non-Hodgkin's Lymphoma.

The present invention not only relates to therapeutic treatment of those suffering from any of the above cancers but also to treatment of relapses of these cancers as well as preventive treatment, e.g. treatments of patients being predisposed or susceptible to the disease.

"Non-malignant disease" as used herein relates to any disease not falling under the above definition given for cancers. Non-malignant diseases which may be treated with the liquid pharmaceutical formulation or the HCLF of the present invention include a disease selected from the group consisting of Autoimmune Diseases, Blood Coagulation Disorders, Blood Platelet Disorders, Blood Protein Disorders, Hematologic Diseases, Hemorrhagic Disorders, Hemostatic Disorders, Lymphatic Diseases, Purpura, Thrombocytopenia, Thrombotic Microangiopathies, Haemostatic Disorders, Vascular Diseases, Rheumatic Diseases, Connective Tissue Diseases, Herpesviridae Infections and DNA Virus Infections. In particular, the liquid pharmaceutical formulation or the HCLF of the present invention may be used for the treatment of autoimmune diseases.

"Autoimmune diseases" or "autoimmune related conditions" as used herein relates to any disease involving an inappropriate immune response of the body against tissues and/or substances naturally present in the body. Autoimmune diseases or autoimmune related conditions include arthritis (e.g. rheumatoid arthritis, juvenile rheumatoid arthritis, osteoarthritis, psoriatic arthritis), psoriasis, dermatitis (e.g. atopic dermatitis), chronic autoimmune urticaria, polymyositis/dermatomyositis, toxic epidermal necrolysis, systemic scleroderma and sclerosis, inflammatory bowel disease (e.g. Crohn's disease, ulcerative colitis), infant respiratory distress syndrome, adult respiratory distress syndrome (ARDS), meningitis, allergic rhinitis, encephalitis, uveitis, colitis, glomerulonephritis, allergic conditions, eczema, asthma, conditions involving infiltration of T cells and chronic inflammatory responses, atherosclerosis, autoimmune myocarditis, leukocyte adhesion deficiency, systemic lupus erythematosus (SLE), lupus (e.g. nephritis, non-renal, discoid, alopecia), juvenile onset diabetes, multiple sclerosis, allergic encephalomyelitis, immune responses associated with acute and delayed hypersensitivity mediated by cytokines and T-lymphocytes, tuberculosis, sarcoidosis, granulomatosis including Wegener's granulomatosis, agranulocytosis, vasculitis (including ANCA), aplastic anemia, Coombs positive anemia, Diamond Blackfan anemia, immune hemolytic anemia including autoimmune hemolytic anemia (AIHA), pernicious anemia, pure red cell aplasia (PRCA), Factor VIII deficiency, hemophilia A, autoimmune neutropenia, pancytopenia, leukopenia, diseases involving leukocyte diapedesis, CNS inflammatory disorders, multiple organ injury syndrome, myasthenia gravis, antigen-antibody complex mediated diseases, anti-glomerular basement membrane disease, anti-phospholipid antibody syndrome, allergic neuritis, Bechet disease, Castleman's syndrome, Goodpasture's Syndrome, Lambert-Eaton Myasthenic Syndrome, Reynaud's syndrome, Sjorgen's syndrome, Stevens-Johnson syndrome, solid organ transplant rejection (including pretreatment for high panel reactive antibody titers,

IgA deposit in tissues, etc), graft versus host disease (GVHD), pemphigoid bullous, pemphigus (all including vulgaris, foliatis), autoimmune polyendocrinopathies, Reiter's disease, stiff-man syndrome, giant cell arteritis, immune complex nephritis, IgA nephropathy, IgM polyneuropathies or IgM mediated neuropathy, idiopathic thrombocytopenic purpura (ITP), thrombotic throbocytopenic purpura (TTP), autoimmune thrombocytopenia, autoimmune disease of the testis and ovary including autoimune orchitis and oophoritis, primary hypothyroidism; autoimmune endocrine diseases including autoimmune thyroiditis, chronic thyroiditis (Hashimoto's Thyroiditis), subacute thyroiditis, idiopathic hypothyroidism, Addison's disease, Grave's disease, autoimmune polyglandular syndromes (or polyglandular endocrinopathy syndromes), Type I diabetes also referred to as insulin- dependent diabetes mellitus (IDDM) and Sheehan's syndrome; autoimmune hepatitis, Lymphoid interstitial pneumonitis (HIV), bronchiolitis obliterans (non-transplant) vs NSIP, Guillain-Barre'Syndrome, Large Vessel Vasculitis (including Polymyalgia Rheumatica and Giant Cell (Takayasu's) Arteritis), Medium Vessel Vasculitis (including Kawasaki's Disease and Polyarteritis Nodosa), ankylosing spondylitis, Berger's Disease (IgA nephropathy), Rapidly Progressive Glomerulonephritis, Primary biliary cirrhosis, Celiac sprue (gluten enteropathy), Cryoglobulinemia, ALS, coronary artery disease. In particular, the liquid pharmaceutical formulation or the HCLF is used for the treatment of a disease selected from the group consisting of arthritis, thrombocytopenia purpura and/or systemic lupus erythematosum. In one embodiment, the liquid pharmaceutical formulation is used for the treatment of arthritis, optionally rheumatoid arthritis. In another embodiment, the liquid pharmaceutical formulation is used for the treatment of thrombocytopenia purpura. In a further embodiment, the liquid pharmaceutical formulation is used for the treatment of systemic lupus erythematosum.

"Inflammatory disease" as used herein denotes any disease involving acute or chronic inflammation, including inflammatory disorders such as allergies, asthma, cancers and autoimmune diseases.

In one embodiment, the liquid pharmaceutical formulation or the HCLF of the invention may be for use in the treatment of a disease selected from the group consisting of leukemia, lymphoma, and/or autoimmune diseases.

In another embodiment, the liquid pharmaceutical formulation or the HCLF of the invention may be for use in the treatment of a disease selected from the group consisting of Burkitt Lymphoma, Epstein-Barr Virus Infections, B-Cell Leukemia, Chronic Lymphocytic B-Cell Leukemia, Acute Lymphoblastic Leukemia, Lymphoid Leukemia, Prolymphocytic Leukemia, Hairy Cell Leukemia, Multiple Myeloma, B-Cell Lymphoma, Marginal Zone B-Cell Lymphoma, Follicular Lymphoma, Diffuse Large B-Cell Lymphoma, Immunoblastic Large-Cell Lymphoma, Mantle-Cell Lymphoma, Non-Hodgkin Lymphoma, Lymphomatoid Granulomatosis, Plasma Cell Neoplasms, Precursor Cell Lymphoblastic Leukemia-Lymphoma, Tumor Virus Infections, Waldenstrom Macroglobulinemia, Rheumatoid Arthritis, Immunoproliferative Disorders, Prolymphocytic Lymphoma, Diffuse Large B-Cell Lymphoma, Immunoblastic Large-Cell Lymphoma, Mantle-Cell Lymphoma, Lymphomatoid Granulomatosis, Lymphoproliferative Disorders, Paraproteinemias, Precursor Cell Lymphoblastic Leukemia-Lymphoma, Thrombocytopenic Purpura, Idiopathic Thrombocytopenic Purpura, Blood Coagulation Disorders, Blood Platelet Disorders, Blood Protein Disorders, Hematologic Diseases, Hemorrhagic Disorders, Hemostatic Disorders, Lymphatic Diseases, Purpura, Thrombocytopenia, Thrombotic Microangiopathies, Haemostatic Disorders, Vascular Diseases, Rheumatoid Arthritis, Rheumatic Diseases, Connective Tissue Diseases, Herpesviridae Infections, and/or DNA Virus Infections.

The liquid pharmaceutical formulations or the HCLF of the invention may be administered to a patient subcutaneously or by other parenteral routes. Other parenteral routes include administration by intravenous, intradermal, intramusclar, intramammary, intraperitoneal, intrathecal, retrobulbar, intrapulmonary injection and/or surgical implantation at a particular site.

However, subcutaneous administration is particularly desirable. One advantage provided by subcutaneous injections is, that it may be performed in short time, in particular when compared to intravenous injection (e.g. approximately 10 minutes for subcutaneous administration compared to about an hour for intravenous infusion). Another advantage is, that while intravenous administration requires an intravenous access which has to be established by trained personnel, subcutaneous injections may even performed by the patient himself, e.g. by using automatic injection devices, thus rendering the therapy more convenient for the patient.

Subcutaneous (SC) administration may be performed via a syringe, optionally a prefilled syringe, an injector pen, optionally an autoinjector pen, an injection device or an infusion pump or a suitable needleless device. Subcutaneous administration may be performed at a single site of the body or at different sites of the body, e.g. at sites adjacent to each other. Suitable sites for subcutaneous administration are known to the person skilled in the art and include, e.g. the thighs or the upper arms.

Usually subcutaneous injections are limited to a volume of approximately 2 ml or less than 2 ml. This is due to viscoelastic tissue resistance and backpressure generated upon injection as well as pain perceived by the patient. Hence, as only a small volume of the antibody formulation can be provided with a single subcutaneous injection, it can be advantageous to have a formulation comprising at least 160 mg/mL of the anti-CD 20 antibody such as a veltuzumab antibody. This may allow administering high doses of antibodies in a small liquid volume suitable for subcutaneous injection.

The liquid pharmaceutical formulation or the HCLF of the invention may be provided in unit dosage form (e.g. in ampoules or prefilled syringe or an injector pen) or in multiple dosage form (e.g. in multi-dose containers or infusion pumps).

The liquid pharmaceutical formulation or the HCLF of the invention may be administered alone or in combination with any further therapeutic agent considered suitable by the person skilled in the art for the treatment of any of the above mentioned diseases. The further therapeutic agent may be administered separately, concurrently or sequentially with the formulation according to the present invention. Examples of such further therapeutic agents are e.g. cytotoxic agents, anti-angiogenic agents, corticosteroids, antibodies, chemotherapeutics, hormones, anti-inflammatory drugs and immunomodulators.

Examples of such therapeutic agents which may be administered alone or in combination with the liquid pharmaceutical formulation can be selected from the group comprising antimitotic, antikinase, alkylating, antimetabolite, antibiotic, alkaloid, antiangiogenic, apoptotic agents and combinations thereof. Chemotherapeutic drugs, for example, can be selected from the group comprising drugs such as vinca alkaloids, anthracyclines, epidophyllotoxin, taxanes, antimetabolites, alkylating agents, antikinase agents, antibiotics, Cox-2 inhibitors, antimitotics, antiangiogenic and apoptotoic agents, particularly doxorubicin, methotrexate, taxol, CPT-II, camptothecans, and others from these and other classes of anticancer agents.

Further examples of such therapeutic agents are described e.g. in the international patent application WO2003/068821, whose content is incorporated herein and referenced herewith.

The invention is further described in the following examples which are solely for the purpose of illustrating specific embodiments of the invention, and are also not to be construed as limiting the scope of the invention in any way.

### Examples

### Example 1

### Preparation of Highly Concentrated Liquid Formulations

A bulk drug substance of veltuzumab is concentrated and diafiltered via tangential flow filtration (TFF) into the final buffer system. An exemplary bulk drug substance comprises 60 g/L veltuzumab, 10 mM histidine, 120 mM sucrose at a pH of 5.5 as depicted in figure 2. The final bulk drug substance was then sterile filtered and stored below -40 °C.

### The excipients used in the invention were generally of high purity and quality complying to compendial specifications (e.g. Pharmacopoeia Europea)

The preparation of the high concentrated liquid formulation was performed via tangential flow filtration with a 30 kDa membrane. First, the buffer of the final bulk was exchanged against the new formulation buffer systems according to the invention. This diafiltration (DF) step is exemplarily shown in figure 2. Usually, an 8 fold volume exchange to remove the original buffer was performed. In the next step (UF, ultrafiltration), the solution is concentrated to approximately 285 g/L of veltuzumab. The filtration unit is then flushed with diafiltration buffer to minimize losses. The concentration after flushing is approx. 200 g/L. The remaining excipients were then added as stock solutions. The final concentration was adjusted to 160 g/L by dilution with formulation buffer which is depicted in figure 2 under mAb Drug Product Pool.

All formulations at the stage of the Drug Product Pool were then sterile filtered through a filter of pore size of 0.2 µm and filled aseptically into 2 mL glass vials. The vials were stoppered with ETFE coated rubber stoppers and sealed with aluminum crimp caps.

The different formulations were subjected to various stress conditions: For example elevated temperature (25 and 40 °C), mechanical stress (24 h shaking) and freeze-thaw stress. Furthermore all samples were put on long-term stability at refrigerated temperature (2-8 °C)

The samples were analyzed by the following analytical methods before and after applying the stress tests

**Table 1 Analytical method overview**

| **Parameter** | **Method** |
|---|---|
| Content or Assay | Size exclusion chromatography (SEC) |
| Purity (monomer content, aggregates, fragments) | Size exclusion chromatography (SEC) |
| Veltuzumab active concentration | Surface Plasmone Resonance (SPR), Biacore |
| Purity (aggregates, fragments) | SDS-PAGE |
| Product Charge Heterogeneity | Cation Exchange Chromatography (CEX) |
| Sub-visible Particles | Micro Flow Imaging (MFI) |
| Potency | Cell based CDC assay |
| Opalescence / Turbidity | Visual against reference suspensions |

Further exemplary methods for providing a high concentration liquid formulation of an antibody according to the present invention are depicted in figures 1 and 3.

### Example 2

### Comparison of two formulations with high concentrated liquid formulation of veltuzumab: Formulation A (phosphate-citrate-mannitol buffer) with Formulation B (histidine-sorbitol buffer)

The following formulations were compared after three months storage at 25 °C:

Formulation A: 150 mg/mL veltuzumab, Mannitol 12mg/mL, sodium chloride 6.2 mg/mL, disodium hydrogen phosphate heptahydrate 2.3 mg/mL, sodium dihydrogen phosphate monohydrate 0.76 mg/mL, citric acid monohydrate 1.3 mg/mL, sodium citrate dihydrate 0.34 mg/mL, polysorbate 80 1.0 mg/mL, pH 5.2

Formulation B: 150 mg/mL veltuzumab, sorbitol 50 mg/mL, L-histidine 30 mM, polysorbate 20 0.1 mg/mL, acetic acid q. s., pH 5.2

**Table 2 Comparision of Fomrulation A and B after three months storage at 25 °C**

| **Parameter (method)** | **Formulation A** | **Formulation B** |
|---|---|---|
| Assay (SEC) in mg/mL | 142,9 | 146,7 |
| Aggregates (SEC) in % | 1,91 | 1,23 |
| Fragments (SEC) in % | 2,59 | 1,38 |
| Sub-visible Particles (MFI) | 18 ≥ 10 µm 6≥25 µm | 35 ≥10 µm 4 ≥ 25 µm |
| Active concentration (SPR) in mg/mL | 123,6 | 140,2 |
| Potency (CDC) in % of Reference Standard | 88,0 | 95,5 |

As can be derived from table 2, formulation B which is a formulation according to this invention comprising a histidine buffer system is clearly superior to Formulation A, especially considering the parameters aggregation, fragmentation and active concentration. Assay, sub-visible particles and potency are within comparable ranges.

It was thus clearly shown, that histidine does not only serve as a buffer substance but also stabilizes veltuzumab at higher concentrations.

### Example 3

### Formulation optimization with regard to histidine and pH

Formulation B as defined in Example 2 was optimized using long term storage conditions.

In particular, the following two factors were investigated: histidine concentration (from 10 to 50 mM) and pH (from 4.8 to 6.2).

The responses were the analytical parameters as shown in table 5. A full factorial statistical design of experiments using the software Modde (Umetrics) was used. Aggregation proved to be the only analytical parameter (response) that was significantly influenced by the two factors histidine concentration and pH. All other responses were not influenced by the factors within the experimental range.

In figure 4 the response contour plot for aggregation is depicted. At rather low pH and high histidine concentration aggregation tendency can be slowed down. However, it could be shown in another set of experiments (not shown here) that histidine concentrations higher than 50 mM (evaluated up to 100 mM) did not reduce aggregation any further. It has further to be considered that for subcutaneous formulations the pH should be close to the physiologic pH of 7.4. The concentration of sorbitol can be adjusted accordingly to provide physiologic tonicity of the solution.

### Example 4

### Formulation optimization with regard to polysorbate 20 concentration

It is generally known that protein solutions tend to form insoluble aggregates upon mechanical stress. This phenomenon is commonly explained as surface induced protein degradation and can be reduced by the introduction of non-ionic surface active ingredients such as polysorbates into the formulation.

Formulation C (Veltuzumab 160 g/L, sorbitol 40 g/L, L-histidine 30 mM, pH 5.5) was used in these examples to determine which concentration of polysorbate 20 would be needed to stabilize the liquid formulation against particle formation upon mechanical stress and freeze-thaw stress. Four levels ofpolysorbate 20 were tested: 0; 0.1, 0.2 and 0.3 g/L.

**Table 3 Formulation C and various polysorbate 20 concentrations**

| | |
|---|---|
| Veltuzumab | 160 mg/mL |
| sorbitol | 40 mg/mL. |
| L-Histidine | 30 mM |
| pH | 5.5 |
| Polysorbate 20 | 0; 0.1; 0.2; 0.3 mg/mL |

The results are summarized in the following table:

**Table 4 Comparison of Formulation C with different levels of Polysorbate 20**

| **Freeze-Thaw (4 cycles)** | | | | |
|---|---|---|---|---|
| PS 20 conc. | 0 mg/mL | 0.1 mg/mL | 0.2 mg/mL | 0.3 mg/mL |
| Assay (SEC) in mg/mL | 165.4 | 164.5 | 167.0 | 166.4 |
| Active concentration (SPR) in mg/mL | 157.9 | 156.4 | 155.1 | 148.8 |
| Aggregates (SEC) in % | 0.38 | 0.38 | 0.40 | 0.39 |
| Fragments (SEC) in % | 1.19 | 1.25 | 1.27 | 1.27 |
| Particles (MFI) | 310≥10 µm 24 ≥ 25 µm | 24 ≥ 10 µm 8 ≥ 25 µm | 107 ≥ 10 14 ≥ 25 µm | 26 ≥ 10 µm 4≥25µm |
| Potency (BP) in % of Reference Standard | 92 | 96 | 81 | 96 |

| **Shaking (24 h)** | | | | |
|---|---|---|---|---|
| PS 20 conc. | 0 mg/mL | 0.1 mg/mL | 0.2 mg/mL | 0.3 mg/mL |
| Assay (SEC) in mg/mL | 166.7 | 166 | 166.3 | 165.4 |
| Active concentration (SPR) in mg/mL | 157.8 | 158.2 | 152.3 | 153.7 |
| Aggregates (SEC) in % | 0.42 | 0.40 | 0.41 | 0.40 |
| Fragments (SEC) in % | 1.25 | 1.23 | 1.25 | 1.29 |
| Particles (MFI) | 37000 ≥ 10 µm 5800 ≥ 25 µm | 2500 ≥ 10 µm 139 ≥ 25 µm | 97 ≥ 10 µm 8 ≥ 25 µm | 77 ≥10 µm 8 ≥ 25 µm |
| Potency (BP) in % of Reference Standard | 92 | 89 | 81 | 85 |

| **12 months storage at 2-8 °C** | | | | |
|---|---|---|---|---|
| PS 20 conc. | 0 mg/mL | 0.1 mg/mL | 0.2 mg/mL | 0.3 mg/mL |
| Assay (SEC) in mg/mL | 163.0 | 163.0 | 162.4 | 163.9 |
| Active concentration (SPR) in mg/mL | 155.3 | 159.8 | 163.5 | 163.0 |
| Aggregates (SEC) in % | 0.53 | 0.51 | 0.54 | 0.52 |
| Fragments (SEC) in % | 1.21 | 1.18 | 1.13 | 1.13 |
| Potency (BP) in % of Reference Standard | 90 | 90 | 94 | 100 |

As expected, the addition of polysorbate 20 has great influence on the formation of (sub-visible) particles determined by Micro Flow Imaging (MFI). After freeze-thaw the particle counts are only slightly elevated and 0.1 mg/mL of polysorbate 20 would be sufficient to prevent particle formation. However, after shake stress the particle counts are much higher. The addition of 0.1 mg/mL of polysorbate 20 is not sufficient to completely avoid sub-visible particles in the solution.

The four formulation with different levels ofpolysorbate 20 were also put on long term storage conditions (2-8 °C) to verify the compatibility of veltuzumab with the surfactant during normal storage. After 12 months storage evidently there is no influence of the investigated polysorbate levels on any of the tested quality attributes (parameters). Therefore, 0.2 mg/mL of polysorbate 20 seems to be the ideal amount to stabilize veltuzumab against particle formation.

### Example 5

### Treatment of patients with the formulation

A randomized, double blind, placebo controlled, multicentre, multinational phase II, 4-arm parallel group trial in subjects with moderate to severe rheumatoid arthritis (RA) insufficiently controlled with methotrexate (MTX) or methotrexate plus antitumour necrosis factor (anti-TNF) biological treatment, comparing three different subcutaneous (s.c.) dosages of anti-CD20 monoclonal antibody veltuzumab to placebo as an add-on therapy to methotrexate is conducted. 400 subjects are screened to allow 320 eligible subjects to be randomised to four treatment arms in a 1:1:1:1 ratio (80 subjects per arm).

Veltuzumab is a humanised monoclonal IgG1 antibody which targets the CD20 epitope that is widely expressed on the surface of mature B-cells leading to their depletion. Veltuzumab is administered by s.c. injection without steroid premedication and is expected to offer improved convenience and cost effectiveness compared to other anti-CD20 therapies.

The trial is designed to compare three different dose levels (160 mg, 320 mg and 640 mg) of veltuzumab to placebo, administered weekly for four weeks (Days 1, 8, 15 and 22) by s.c. injection to subjects with moderate to severe RA (cumulative veltuzumab doses 640 mg, 1280 mg, and 2560 mg respectively). All subjects are continued on stable co-medication with methotrexate. The primary end-point, the American College of Rheumatology 20 (ACR20) response rate, is evaluated at Week 24.

Veltuzumab s.c. is expected to be generally well tolerated. No clinically significant observations or treatment-related serious adverse events are expected.

The total volume administered s.c. to an individual patient is 2.0 mL per injection. In conclusion, subcutaneous veltuzumab is expected to be delivered quickly, comfortably and safely to patients. The patient experience is expected to be favourable, which is support testing of veltuzumab in Phase III trial.

## Claims

1. A method for preparation of a high concentration liquid formulation of an antibody having a concentration C^{H} of the antibody, comprising the steps of:
a) providing a solution containing the antibody in a starting concentration C^{S};
b) ultrafiltering the solution of step (a) in order to obtain a solution having an intermediate concentration C^{I} of the antibody, wherein C^{I} is at least about 260 mg/mL; and
c) diluting the solution of step (b) to a concentration C^{H} of the antibody in order to obtain the high concentration liquid formulation.

2. The method according to claim 1, wherein the solution of step (a) further contains a buffering agent which is optionally an amino acid, such as histidine.

3. The method according to claim 1 or 2, further comprising between step (a) and step (b) a step of diafiltering the solution of step (a) with a buffer solution, wherein the buffering agent is optionally an amino acid, such as histidine.

4. The method according to any of the preceding claims, wherein the solution which is subjected to ultrafiltering in step (b) contains 40 mM histidine and has a pH value of 5.45.

5. The method according to any of the preceding claims, wherein the solution which is subjected to ultrafiltering in step (b) is essentially free of or does not contain a tonicity modifying agent such as sucrose.

6. The method according to any of the preceding claims, wherein the solution which is subjected to ultrafiltering in step (b) is essentially free of or does not contain a surfactant such as polysorbate.

7. The method according to any of the preceding claims, wherein the antibody is an anti-CD20 antibody and/or an IgG antibody, optionally the antibody is a veltuzumab antibody.

8. The method according to any of the preceding claims, wherein the high concentration liquid formulation has an antibody concentration C^{H} of at least 155 mg/mL.

9. The method according to any of the preceding claims, wherein step (b) is performed in an ultrafiltration device and further comprising between step (b) and step (c) a step of flushing the ultrafiltration device with a buffer solution, wherein the buffering agent is optionally an amino acid, such as histidine.

10. A method for stabilizing an anti-CD20 antibody or a fragment thereof in a liquid pharmaceutical formulation in a concentration of at least 155 mg/mL by combining the antibody or fragment thereof which has not been freeze-dried with an aqueous solution comprising an amino acid, optionally histidine.

11. The method according to claim 7, wherein the anti-CD20 antibody is a veltuzumab antibody.

12. Liquid pharmaceutical formulation of a veltuzumab antibody or a fragment thereof comprising at least 155 mg/mL veltuzumab antibody or a fragment thereof and an amino acid, optionally histidine.

13. The liquid pharmaceutical formulation according to claim 12, wherein the concentration of the veltuzumab antibody or a fragment thereof is at least 175 mg/mL, preferably at least 190 mg/mL.

14. The liquid pharmaceutical formulation to claim 12 or 13, wherein the concentration of the veltuzumab antibody or a fragment thereof is at least 200 mg/mL, preferably at least 220 mg/mL.

15. The liquid pharmaceutical formulation according to any one of claims 12 to 14, wherein the concentration of the amino acid, optionally histidine, is in the range of 1 mM to 100 mM.

16. The liquid pharmaceutical formulation according to claim 15, wherein the concentration of the amino acid, optionally histidine, is in the range of 10 mM to 60 mM, optionally in the range of 20 mM to 50 mM.

17. The liquid pharmaceutical formulation according to any of claims 12 to 16, further comprising a surfactant, optionally a nonionic surfactant.

18. The liquid pharmaceutical formulation according to claim 17, wherein the surfactant, optionally the nonionic surfactant, is a polysorbate, optionally polysorbate 20 or polysorbate 80.

19. The liquid pharmaceutical formulation according to claim 17 or 18, wherein the surfactant, optionally the nonionic surfactant, is present in a concentration of at least 0.01 mg/mL, optionally in the range of 0.1 mg/mL to 1 mg/mL.

20. The liquid pharmaceutical formulation according to any of claims 12 to 19, further comprising a tonicity modifying agent.

21. The liquid pharmaceutical formulation according to claim 20, wherein the tonicity modifying agent is sorbitol and/or mannitol, optionally the concentration of the tonicity modifying agent is in the range of 5.5 mM to 550 mM.

22. The liquid pharmaceutical formulation according to any of claims 12 to 21 having a pH value in the range of 4.8 to 7.0, optionally a pH value of 5.5 ± 0.3.

23. The liquid pharmaceutical formulation according to claim 12 comprising
a) at least 160 mg/mL veltuzumab antibody or a fragment thereof;
b) 220 mM sorbitol;
c) 30 mM histidine;
d) 0.2 mg/mL polysorbate 20; and
having a pH value in the range of 5.0 to 6.0, optionally 5.5±0.3.

24. The liquid pharmaceutical formulation according to any of claims 12 to 23 for use as a medicament.

25. The liquid pharmaceutical formulation according to claim 24, wherein the medicament is for subcutaneous administration.

26. The liquid pharmaceutical formulation according to any of claims 12 to 25, for use in the treatment of cancer or a non-malignant disease, optionally an inflammatory or autoimmune disease.

27. The liquid pharmaceutical formulation according to any of claims 12 to 26, for use in the treatment of a disease selected from the group consisting of Burkitt Lymphoma, Epstein-Barr Virus Infections, B-Cell Leukemia, Chronic Lymphocytic B-Cell Leukemia, Acute Lymphoblastic Leukemia, Lymphoid Leukemia, Prolymphocytic Leukemia, Hairy Cell Leukemia, Multiple Myeloma, B-Cell Lymphoma, Marginal Zone B-Cell Lymphoma, Follicular Lymphoma, Diffuse Large B-Cell Lymphoma, Immunoblastic Large-Cell Lymphoma, Mantle-Cell Lymphoma, Non-Hodgkin Lymphoma, Lymphomatoid Granulomatosis, Plasma Cell Neoplasms, Precursor Cell Lymphoblastic Leukemia-Lymphoma, Tumor Virus Infections, Waldenstrom Macroglobulinemia, Rheumatoid Arthritis, Immunoproliferative Disorders, Prolymphocytic Lymphoma, Diffuse Large B-Cell Lymphoma, Immunoblastic Large-Cell Lymphoma, Mantle-Cell Lymphoma, Lymphomatoid Granulomatosis, Lymphoproliferative Disorders, Paraproteinemias, Precursor Cell Lymphoblastic Leukemia-Lymphoma, Thrombocytopenic Purpura, Idiopathic Thrombocytopenic Purpura (ITP), Blood Coagulation Disorders, Blood Platelet Disorders, Blood Protein Disorders, Hematologic Diseases, Hemorrhagic Disorders, Hemostatic Disorders, Lymphatic Diseases, Purpura, Thrombocytopenia, Thrombotic Microangiopathies, Haemostatic Disorders, Vascular Diseases, Systemic lupus erythematosus (SLE), Multiple sclerosis, Rheumatic Diseases, Juvenile rheumatoid arthritis, Osteoarthritis, Psoriatic arthritis, Psoriasis, inflammatory bowel disease, such as Crohn's disease, ulcerative colitis, Connective Tissue Diseases, Herpesviridae Infections, and/or DNA Virus Infections.
